# EUROPEAN PATENT APPLICATION

(11) **EP 4 624 454 A1**
(43) Date of publication of application: **01.10.2025**
(21) Application number: 23894041.5
(22) Date of filing: 27.11.2023
(51) Int. Cl.: C07D 209/20, C07D 233/64, A61P 39/06, A61P 25/16, A61P 25/28, A61P 43/00, A61K 31/405, A61K 31/4172

(54) **NON-COVALENT DIMER CATION, AND SALT THEREOF, PREPARATION METHOD THEREFOR AND USE THEREOF**

(30) Priority: 25.11.2022 CN 202211494009
(71) Applicant: Suzhou Protonation Chain Biotechnology Co., Ltd, Suzhou, Jiangsu 215011 (CN)
(72) Inventor: ZHAO, Zizhen, Suzhou, Jiangsu 215011 (CN); WANG, Zhigang, Suzhou, Jiangsu 215011 (CN)
(74) Representative: Schulz Junghans Patentanwälte PartGmbB
(86) International application number: PCT/CN2023/134364
(87) International publication number: WO 2024/109952

(57) **Abstract**

Provided is a non-covalent dimer cation of the present application, which is a cation as shown in the drawing of the abstract, a tautomer thereof, or a stereoisomer thereof. Provided is a salt of the present application, comprising a cation and a first anion of the present application. Provided is the use of the cation of the present application or the salt of the present application, for example, the use in the preparation of a drug for promoting cell repair or cell proliferation or wound repair.

## Description

### FIELD

The present application relates to the field of biological medicines, and more specifically, to a non-covalent dimer cation, a salt thereof, a method for preparing thereof, and use thereof in cell repair, or cell proliferation or wound repair, or other aspects.

### BACKGROUND

The ability of cell repair plays an important role in living organisms. Many factors may cause cell damage, which in turn leads to tissue and organ damages and hence to symptoms, and the ability of cell self-repair is particularly important. A number of studies have shown that young cells have a stronger ability and willingness to repair themselves than older cells, that is, young cells are more prone to repair themselves rather than waiting for immune cells to process, and accordingly, young cells have a stronger repair ability.

The ability of cell repair also varies significantly according to different cells. Cells of various glandular organs, such as liver, pancreas, endocrine glands, sweat glands, sebaceous glands and renal tubular epithelial cells, exhibit strong repair ability when they are damaged internally or externally. Cells with weak or no repair ability, such as central nervous cells and ganglion cells, are extremely difficult to restore their original functions after being damaged. Cardiac muscle cells are replaced by fibrous connective tissues after being damaged and thus very difficult to restore their original structures and functions due to extremely weak repair ability.

The occurrence of many diseases is also closely related to the decline or deficiency of the ability of cell repair, and the probability of certain diseases in the elderly and those exposed to risk is greatly increased, which is due to the decline or deficiency of cell repair levels in their bodies. For example, neurodegenerative diseases such as Parkinson's disease and Alzheimer's disease that are suffered by the elderly, gastric mucosal damage, gastric ulcers and the like that occur in people who are heavy drinkers over a long period of time, or some organ diseases caused by excessively large pressure of organ functions, are all caused by the insufficient ability of cell repair.

Studies have shown that in the vast majority of damaged cells, there is usually a higher level of oxidative stress. That is, when the cells are damaged, they reflect an abnormal state, which is due to damages or damage repair, and a higher level of ROS will occur, thereby inhibiting a self-repair function of cells, and further worsening the diseases. Therefore, reducing an intracellular ROS level is also an effective means of restoring the ability of cell repair, but this method does not work directly, resulting in poor effect.

There are many factors that affect the ability of cell repair, including the types, degrees and extents of damages, the age of individuals, the nutritional statuses of organisms, or the effects of drugs. However, the current clinical stage in the treatment of a related disease still focuses on the "targeted" treatment of the disease, while ignoring the repair ability of the cells themselves, so the progress in the treatment means of some diseases is slow, and due to side effects of some drugs, the phenomenon of prioritizing one aspect while neglecting another often occurs.

Cell proliferation, as one of the important physiological functions of living cells, is an important life characteristic of living organisms. The proliferation of cells is the basis for the growth, development, reproduction, and heredity of living organisms. Cell proliferation is also an important life characteristic of living organisms, and human cells proliferate in a dividing manner to replenish aging or dead cells in the body.

Cell proliferation is a complex and vital life activity, which undergoes countless cell proliferations from the development of a fertilized egg into an independent individual. Each organ also undergoes regulated cell proliferation all the time to maintain its physiological life activities. When invaded by pathogens, immune cells proliferate to protect the individual's health. When an individual suffers physical wounds, the corresponding cells will also start to proliferate in order to repair tissues or organ damages.

Wounds refer to the destruction of human tissues or organs caused by mechanical factors. It is extremely common and can occur not only during times of war but also in peacetime. Wounds are of a broad concept, which includes: cuts, stabs, bruises, and sprains. Due to the rapid development of industries, agriculture, transportation and sports, the number of wounds caused by various accidents is increasing day by day. The wounds have high incidence and broad influences. Due to the maturity and popularization of internal medicine and surgery, surgical wounds, as a type of wounds, also frequently occur in people's daily lives.

Wound healing refers to a series of complex pathological and physiological processes that occur during external injuries or other diseases, where local tissues are repaired by proliferation or regeneration after tissue defects. After the occurrence of a wound, the body undergoes a highly coordinated healing process, involving a complex network of various cells, cytokines and extracellular matrices. The cells participating in skin wound healing mainly include various inflammatory cells and tissue repair cells, the former including neutrophils, macrophages, etc., and the latter mainly including vascular endothelial cells, epidermal cells, fibroblasts and nerve cells. These series of cells are indispensable for achieving their respective functions through cell proliferation and promoting the healing of wound surfaces.

At present, in clinical treatment, the focus is mainly on performing sterilization and disinfection on wounds for a cleaning purpose. However, as for the specific repair work, it still relies on the body's own coordinated repair ability. Elderly people have a weaker ability of tissue cell proliferation, which leads to slow wound healing, high infection risk, and interference with normal life. In severe cases, the inflammations caused by infections can even threaten the life. However, when the proliferation rate of healed tissue cells is slow, a relatively hypertrophic connective tissue will be formed. This connective tissue is difficult to integrate with the original skin, thus presenting as a scar, which affects the appearance. Therefore, some studies suggest that accelerating the wound healing process may facilitate reducing the formation of scars.

### SUMMARY

The present application provides a non-covalent dimer cation. The non-covalent dimer cation is a cation of formula (A), a tautomer thereof, or a stereoisomer thereof, wherein a bond "-------" in formula (A) is a non-covalent bond; R⁷ is selected from a group consisting of hydrogen, hydroxymethyl, -C-R⁶, and -C(=O)-R⁶; R¹ is selected from a group consisting of hydrogen, hydroxyl, sulfhydryl, amino, methyl, ethyl, and phenyl; Q¹ is selected from a group consisting of hydrogen, hydroxyl, sulfhydryl, amino, methyl, ethyl, phenyl, chloro, cyano, and carboxyl; R² and Q² are each independently selected from a group consisting of hydrogen, methyl, ethyl, propyl, phenyl, benzyloxymethyl, and triphenylmethyl; R³ and Q³ are each independently selected from a group consisting of hydrogen, hydroxyl, sulfhydryl, amino, methyl, ethyl, and phenyl; R⁴ and Q⁴ are each independently selected from a group consisting of hydrogen, methyl, ethyl, and propyl; R⁵ and Q⁵ are each independently selected from a group consisting of hydrogen, benzyl, amino, methylamino, hydrazino, trimethylammonium, pyrrolyl, and amino further linked with a substituent derived from a C-terminal carbon atom of an amino acid, dipeptide or tripeptide; R⁶ and Q⁶ are each independently selected from a group consisting of hydroxyl, deprotonated hydroxyl, methoxyl, methylamino, ethylamino, and a substituent derived from an N-terminal nitrogen atom of an amino acid, dipeptide or tripeptide; and *m* and *n* are each independently selected from a group consisting of 0 and 1.

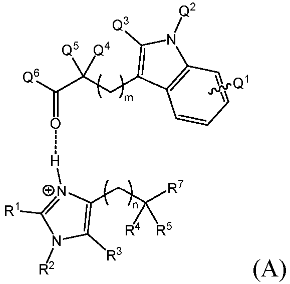

The present application provides a salt. The salt comprises the cation of the present application, and a first anion, the first anion comprising at least one selected from a group consisting of a fluoride ion, a chloride ion, a bromide ion, an iodide ion, a sulfide ion, a nitrate ion, a sulfate ion, a sulfite ion, a thiosulfate ion, a persulfate ion, a selenate ion, a phosphate ion, a carbonate ion, a hexafluorophosphate ion, a hexafluorosilicate ion, an acetate ion, a sulfonate ion, a benzoate ion, and a polyphosphate ion.

The present application provides a method for preparing the cation of the present application or the salt of the present application, comprising: contacting a first reactant with a first acid to obtain a first acid salt of the first reactant; contacting the first acid salt of the first reactant with a first salt comprising a first anion in an acidic condition, to obtain a first solution comprising a protonated first acid salt cation of the first reactant and the first anion; adding a second reactant to the first solution in a strong reducing and acidic condition, to obtain a second solution or a first intermediate; and adding a first base to the second solution or contacting the first base with the first intermediate, the first reactant comprising a compound of formula (C), and the second reactant comprising a compound of formula (D).

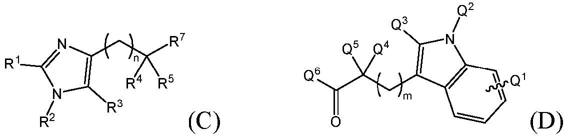

The present application provides use of the cation of the present application or the salt of the present application in preparation of a drug, and in particular in antioxidation, preparation of an antioxidant, preparation of a drug for inhibiting, reducing or reversing oxidative stress in human or animal cells or for inhibiting, reducing or scavenging reactive oxygen species from human or animal bodies, or preparation of a drug for treating a disease or a symptom related to oxidative stress or reactive oxygen species. The present application provides a method for preparing a drug, comprising: preparing the cation of the present application or the salt of the present application.

The present application provides use of the cation of the present application or the salt of the present application in preparation of a drug, and in particular in promotion of cell proliferation, or preparation of a drug for promoting cell proliferation or treating, alleviating or repairing a wound. The present application provides a method for preparing a drug, comprising: preparing the cation of the present application or the salt of the present application.

### BRIEF DESCRIPTION OF THE DRAWINGS

FIG. 1 shows results of reactive oxygen species scavenging experiments of 2-phenyl-4,5-dihydro-4,4,5,5-tetramethyl-1H-imidazolyl-1-oxyl-3-oxide (PTIO) models in some examples of the present application.
FIG. 2 shows cell viability of ethanol-induced damage models of gastric mucosal epithelial cells in some examples of the present application.
FIG. 3 shows cell viability of Parkinson's disease models in some examples of the present application.
FIG. 4 shows cell viability of Alzheimer's disease models in some examples of the present application.
FIG. 5 shows transcriptomic analysis results in some examples of the present application.
FIG. 6 shows cell viability of epidermal fibroblast proliferation experiments in some examples of the present application.
FIG. 7 shows cell viability of keratinocyte proliferation experiments in some examples of the present application.
FIG. 8 shows cell viability of vascular endothelial cell proliferation experiments in some examples of the present application.
FIG. 9 shows cell viability of nerve cell proliferation experiments in some examples of the present application.
FIG. 10 shows transcriptomic analysis results in some examples of the present application.

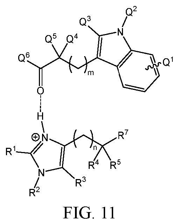

FIG. 11 shows formula (A).

### DETAILED DESCRIPTION OF THE EMBODIMENTS

As used herein, the singular term means one or more. For example, "an element" or "one element" means one or more elements. As used herein, the term "a plurality of" means at least two.

As used herein, the term "about" means approximately, in the range of about or around. When used in combination with a value range, the term "about" modifies the range by extending the limit above or below the value provided. Generally, the term "about" is used herein to give a value plus or minus 10% from the value provided. On the one hand, the term "about" means plus or minus 20% of a value of the number defined by it. For example, "about 50%" means a range of 45% to 55%. Herein, a value range defined by endpoints includes all integers and fractions within the range (for example, "1 to 5" includes 1, 1.5, 2, 2.75, 3, 3.90, 4, and 5). It should also be understood that all the integers and fractions are defined by the term "about".

As used herein, the term "include", "comprise" or "contain", as a non-exclusive or openended term, is intended to mean that a combination (such as a device, a composition or a method) includes listed elements (such as units of the device, components of the composition, or substantive steps of the method), but other elements are not excluded. As used herein, the term "essentially consisting of ..." means, when used for defining a composition or a method, exclusion of other elements that have any material effect on the combination of the stated objectives, but does not exclude other elements that do not materially affect the basic and novel features of the present application. As used herein, unless otherwise described, the term "consisting of ..." means exclusion of the combination of other elements (units, components, substantive steps or the like), and is not intended to exclude trace amounts of unavoidable impurities. Embodiments defined by each of these connection terms fall within the scope of the present application. As a specific embodiment, a disclosed technical solution that is defined by the term "include", "comprise" or "contain" shall be regarded as also disclosing a corresponding technical solution defined by the term "essentially consisting of ..." or "consisting of ...".

As used herein the term "and/or" means and covers any or all possible combination of one or more associated listed items. When used in a list of two or more items, the term "and/or" means that any one of the listed items may be included alone or any combination of two or more of the listed items may be included. For example, if a group, combination, composition or the like is described as including (or containing) components A, B, C and/or D, the composition may include A alone; B alone; C alone; D alone; the combination of A and B; the combination of A and C; the combination of A and D; the combination of B and C; the combination of B and D; the combination of C and D; the combination of A, B, and C; the combination of A, B, and D; the combination of A, C, and D; the combination of B, C, and D; or the combination of A, B, C, and D.

As used herein, the compound or ion of the present application includes a plurality of variable groups. Those of ordinary skill in the art shall recognize that combinations of the groups that are contemplated by the present application are chemically allowed combinations of compounds or ions.

As used herein, the stereochemistry of a chiral center may be defined according to the conventions of those skilled in the art. That is, the wedged bond " 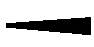 " is used for indicating a group in front of the plane of the paper (oriented towards the viewer), and the hashed bond " 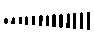 " is used for indicating a group behind the plane of the paper (oriented away from the viewer). It may be understood that such representations are used for indicating a specific single stereoisomer of groups represented by each chemical structure herein. Any bond that is not specifically represented by the wedged bond or the hashed bond herein shall be regarded as not specifically indicating whether the bond is in front of the plane of the paper, or behind the plane of the paper, or located in the plane of the paper. However, it does not exclude that the bond is in front of or behind the plane of the paper if chemically allowed.

As used herein, the term "isomer" refers to compounds with the same molecular formula and different bonding nature or order of atoms or different arrangements of atoms in space. The term "stereoisomer" refers to isomers with different arrangements of atoms in space. The term "enantiomer" refers to stereoisomers with one or more asymmetric centers that are non-superimposable mirror images of each other. The term "diastereomer" refers to stereoisomers that have opposite configurations of one or more asymmetric centers and that do not belong to enantiomers. If a compound has an asymmetric center, for example, if a carbon atom is bonded to four different groups, a pair of enantiomers may exist. A configuration of an enantiomer may be characterized and designated as an R-configuration or an S-configuration by an absolute configuration of one or more asymmetric centers of the enantiomer, or the enantiomer is designated as dextrorotatory or levorotatory based on the manner in which the molecule rotates the plane of polarized light. A chiral compound may exist in the form of an enantiomer alone or a mixture of enantiomers, for example, exist in the form of a racemic mixture. The compound of the present application may contain an asymmetric center or chiral center, and exist in the form of different stereoisomers. It should be regarded that all stereoisomers of the compound of the present application include, but are not limited to, a diastereomer, an enantiomer, an atropisomer, and a mixture thereof, such as a racemic mixture, which form part of the present application.

As used herein, the term "dimer" usually refers to a compound formed by two molecules or ions, such as an amino acid, a peptide, or a derivative or protonated ion thereof, through an interaction, particularly a non-covalent interaction. As used herein, the term "covalent bond" refers to any bond that includes or involves electron sharing. Non-limiting examples of covalent bonds include, but are not limited to, a peptide bond, a glycosidic bond, an ester bond, and a phosphodiester bond. As used herein, the term "non-covalent bond" includes any bond or interaction between two or more moieties that do not include or involve electron sharing. Non-limiting examples of non-covalent bonds or interactions include, but are not limited to, static electricity, π-π effects, the van der Waals force, a hydrogen bond, and the hydrophobic effect, particularly the hydrogen bond.

As used herein, any amino acid with chirality shall be regarded as at least referring to L-amino acid in a case that the chirality is not explicitly described. However, it does not explicitly exclude relevant embodiments of D-amino acid.

As used herein, in particular, the term "disclose or comprise" as described below includes, but is not limited to, the disclosure of the specification (such as, examples), drawings, or claims of the present application, and includes, but is not limited to, the scope of protection of the claims of the present application, or any one or more embodiments (such as, embodiments defined by the term "in an embodiment" or "in some embodiments" herein).

In order to further describe the technical means adopted by the present application to achieve the intended objective and functions, the following describes specific embodiments, structures, features, and functions of the present application in detail with reference to the accompanying drawings and preferred examples.

### Non-covalent dimer cation of the present application

In an aspect, the present application provides a non-covalent dimer cation.

The present application provides a non-covalent dimer cation, which comprises a moiety of formula (A1) and a moiety of formula (A2). Hydrogen (*N^{π}*-H, that is, H marked with an asterisk in formula (A1), or *N^{τ}*-H under the condition that R² is hydrogen) on an imidazole ring in the moiety of formula (A1) forms a non-covalent bond with a more electronegative or most electronegative atom in the moiety of formula (A2), such as oxygen (that is, O marked with an asterisk in formula (A2)) on the carbonyl group in the moiety of formula (A2).

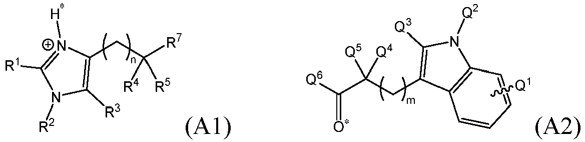

The present application provides a non-covalent dimer cation, which comprises or consists of a cation of formula (A), a tautomer thereof, or a stereoisomer thereof. The bond "-------" in formula (A) is a non-covalent bond.

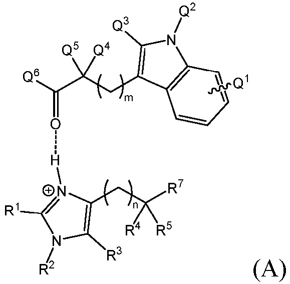

The non-covalent bond, that is, the bond "-------" in formula (A), shall be regarded as comprising all chemically allowed non-covalent bonds that enable the non-covalent dimer cation of the present application to exist stably in its salts, or being consisting of the chemically allowed non-covalent bonds.

According to the understanding of those skilled in the art, the non-covalent bond may be understood as a hydrogen bond. However, through experiments, the applicant has confirmed that the non-covalent bond exists stably in the general condition, for example, in the form of a salt. Particularly, the non-covalent bond can remain stable without being broken at least during determination by mass spectroscopy. That is, the non-covalent dimer cation of the present application can be kept intact during determination by mass spectroscopy, and accordingly an ion peak corresponding to an m/z value of the non-covalent dimer cation of the present application is generated. That is, it should be understood that properties of some hydrogen bonds may be applicable to the non-covalent bond. However, the non-covalent bond does not necessarily comply with all understandings of properties of hydrogen bonds by those skilled in the art, particularly in terms of bond energy. In some embodiments, the non-covalent bond is a bond with bond energy stronger than that of a general NH-:O=C bond, such as a bond with bond energy greater than 8 kJ/mol. 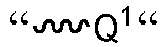 marked in formula (A) and formula (A2) indicates that a substituent Q¹ may be any constituent atom (member) formed on a ring to which it points (i.e., a benzene ring moiety in an indole moiety in each of formula (A) and formula (A2)). Specifically, Q¹ may be formed in position 4, 5, 6, or 7 of the indole moiety in each of formula (A) and formula (A2).

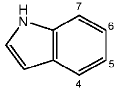

In some embodiments, in the cation of formula (A) or the moiety of formula (A2), Q¹ is located on carbon in position 4 of the indole moiety, as shown in formula (A-4) and formula (A2-4). In some embodiments, in the cation of formula (A) or the moiety of formula (A2), Q¹ is located on carbon in position 5 of the indole moiety, as shown in formula (A-5) and formula (A2-5). In some embodiments, in the cation of formula (A) or the moiety of formula (A2), Q¹ is located on carbon in position 6 of the indole moiety, as shown in formula (A-6) and formula (A2-6). In some embodiments, in the cation of formula (A) or the moiety of formula (A2), Q¹ is located on carbon in position 7 of the indole moiety, as shown in formula (A-7) and formula (A2-7).

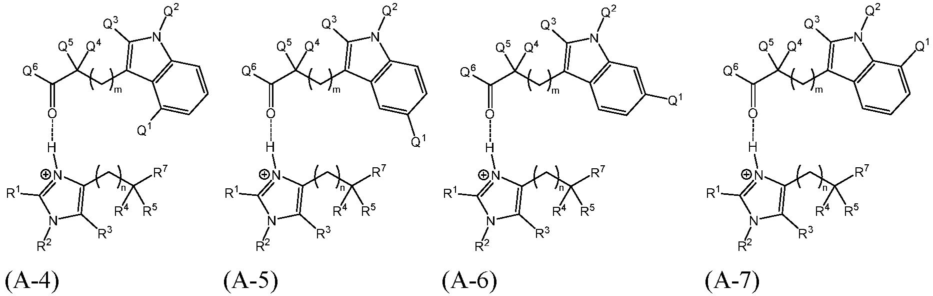

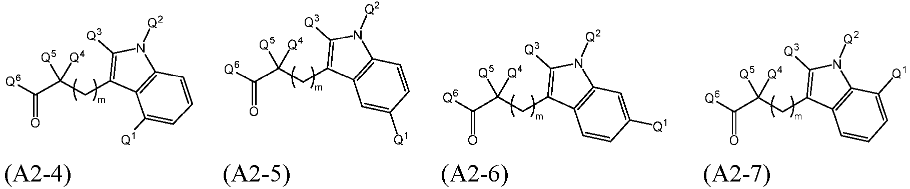

In some embodiments, R⁷ is selected from a group consisting of hydrogen, hydroxymethyl, - C-R⁶, and -C(=O)-R⁶. In some embodiments, R⁷ is hydrogen. In some embodiments, R⁷ is hydroxymethyl. In some embodiments, R⁷ is -C-R⁶. In some embodiments, R⁷ is -C(=O)-R⁶.

In some embodiments, R¹ is selected from a group consisting of hydrogen, hydroxyl, sulfhydryl, amino, methyl, ethyl, and phenyl. In some embodiments, R¹ is selected from a group consisting of hydrogen, hydroxyl, sulfhydryl, amino, methyl, and phenyl. In some embodiments, R¹ is hydrogen. In some embodiments, R¹ is hydroxyl. In some embodiments, R¹ is sulfhydryl. In some embodiments, R¹ is amino. In some embodiments, R¹ is methyl. In some embodiments, R¹ is ethyl. In some embodiments, R¹ is phenyl. In some embodiments, Q¹ is selected from a group consisting of hydrogen, hydroxyl, sulfhydryl, amino, methyl, ethyl, phenyl, chloro, cyano, and carboxyl. In some embodiments, Q¹ is selected from a group consisting of hydrogen, hydroxyl, amino, chloro, cyano, and carboxyl. In some embodiments, Q¹ is hydrogen. In some embodiments, Q¹ is hydroxyl. In some embodiments, Q¹ is sulfhydryl. In some embodiments, Q¹ is amino. In some embodiments, Q¹ is methyl. In some embodiments, Q¹ is ethyl. In some embodiments, Q¹ is phenyl. In some embodiments, Q¹ is chloro. In some embodiments, Q¹ is cyano. In some embodiments, Q¹ is carboxyl.

In some embodiments, R² is selected from a group consisting of hydrogen, methyl, ethyl, propyl, phenyl, benzyloxymethyl, and triphenylmethyl. In some embodiments, R² is selected from a group consisting of hydrogen, methyl, phenyl, and triphenylmethyl. In some embodiments, R² is hydrogen. In some embodiments, R² is methyl. In some embodiments, R² is ethyl. In some embodiments, R² is propyl. In some embodiments, R² is phenyl. In some embodiments, R² is benzyloxymethyl. In some embodiments, R² is triphenylmethyl. In some embodiments, Q² is selected from a group consisting of hydrogen, methyl, ethyl, propyl, phenyl, benzyloxymethyl, and triphenylmethyl. In some embodiments, Q² is selected from a group consisting of hydrogen and methyl. In some embodiments, Q² is hydrogen. In some embodiments, Q² is methyl. In some embodiments, Q² is ethyl. In some embodiments, Q² is propyl. In some embodiments, Q² is phenyl. In some embodiments, Q² is benzyloxymethyl. In some embodiments, Q² is triphenylmethyl.

In some embodiments, R³ is selected from a group consisting of hydrogen, hydroxyl, sulfhydryl, amino, methyl, ethyl, and phenyl. In some embodiments, R³ is selected from a group consisting of hydrogen, sulfhydryl, and methyl. In some embodiments, R³ is hydrogen. In some embodiments, R³ is hydroxyl. In some embodiments, R³ is sulfhydryl. In some embodiments, R³ is amino. In some embodiments, R³ is methyl. In some embodiments, R³ is ethyl. In some embodiments, R³ is phenyl. In some embodiments, Q³ is selected from a group consisting of hydrogen, hydroxyl, sulfhydryl, amino, methyl, ethyl, and phenyl. In some embodiments, Q³ is selected from a group consisting of hydrogen, sulfhydryl, and methyl. In some embodiments, Q³ is hydrogen. In some embodiments, Q³ is hydroxyl. In some embodiments, Q³ is sulfhydryl. In some embodiments, Q³ is amino. In some embodiments, Q³ is methyl. In some embodiments, Q³ is ethyl. In some embodiments, Q³ is phenyl.

In some embodiments, R⁴ is selected from a group consisting of hydrogen, methyl, ethyl, and propyl. In some embodiments, R⁴ is selected from a group consisting of hydrogen and methyl. In some embodiments, R⁴ is hydrogen. In some embodiments, R⁴ is methyl. In some embodiments, R⁴ is ethyl. In some embodiments, R⁴ is propyl. In some embodiments, Q⁴ is selected from a group consisting of hydrogen, methyl, ethyl, and propyl. In some embodiments, Q⁴ is selected from a group consisting of hydrogen and methyl. In some embodiments, Q⁴ is hydrogen. In some embodiments, Q⁴ is methyl. In some embodiments, Q⁴ is ethyl. In some embodiments, Q⁴ is propyl.

In some embodiments, R⁵ is selected from a group consisting of hydrogen, benzyl, amino, methylamino, hydrazino, trimethylammonium, pyrrolyl, and amino further linked with a substituent derived from a C-terminal carbon atom of an amino acid or peptide. In some embodiments, Q⁵ is selected from a group consisting of hydrogen, benzyl, amino, methylamino, hydrazino, trimethylammonium, pyrrolyl, and amino further linked with a substituent derived from a C-terminal carbon atom of an amino acid or peptide. In some embodiments, the amino further linked with a substituent derived from a C-terminal carbon atom of an amino acid or peptide may be represented as "-N-C_{AA}(=O)-Z", where, C_{AA} is the C-terminal carbon atom of the amino acid or peptide, and Z is the remaining moiety of the amino acid or peptide. That is, Z-C_{AA}OOH is an amino acid or peptide, particularly an amino acid, dipeptide or tripeptide. In some embodiments, the amino further linked with a substituent derived from a C-terminal carbon atom of an amino acid or peptide is amino further linked with a substituent derived from a C-terminal carbon atom of an amino acid, dipeptide or tripeptide. In some embodiments, R⁵ is selected from a group consisting of hydrogen, benzyl, amino, methylamino, hydrazino, trimethylammonium, pyrrolyl, and amino further linked with a substituent derived from a C-terminal carbon atom of an amino acid, dipeptide or tripeptide. In some embodiments, Q⁵ is selected from a group consisting of hydrogen, benzyl, amino, methylamino, hydrazino, trimethylammonium, pyrrolyl, and amino further linked with a substituent derived from a C-terminal carbon atom of an amino acid, dipeptide or tripeptide. In some embodiments, the amino further linked with a substituent derived from a C-terminal carbon atom of an amino acid or peptide is selected from a group consisting of acetamido, 2-aminoacetamido (that is, glycylamino, a group formed by further linking a substituent derived from a C-terminal carbon atom of glycine to amino group), 3-aminopropionamido (that is, *β-*alanylamino, a group formed by further linking a substituent derived from a C-terminal carbon atom of *β*-alanine to amino group), and pyroglutamylamino (that is, a group formed by further linking a substituent derived from a C-terminal carbon atom of pyroglutamic acid to amino group). In some embodiments, R⁵ is selected from a group consisting of hydrogen, benzyl, amino, methylamino, hydrazino, trimethylammonium, pyrrolyl, acetamido, 2-aminoacetamido, 3-aminopropionamido, and pyroglutamylamino. In some embodiments, R⁵ is selected from a group consisting of hydrogen, amino, hydrazino, trimethylammonium, pyrrolyl, acetamido, 2-aminoacetamido, 3-aminopropionamido, and pyroglutamylamino. In some embodiments, R⁵ is selected from a group consisting of hydrogen, benzyl, amino, methylamino, hydrazino, pyrrolyl, and acetamido. In some embodiments, R⁵ is hydrogen. In some embodiments, R⁵ is benzyl. In some embodiments, R⁵ is amino. In some embodiments, R⁵ is methylamino. In some embodiments, R⁵ is hydrazino. In some embodiments, R⁵ is trimethylammonium. In some embodiments, R⁵ is pyrrolyl. In some embodiments, R⁵ is amino further linked with a substituent derived from a C-terminal carbon atom of an amino acid or peptide. In some embodiments, R⁵ is acetamido. In some embodiments, R⁵ is 2-aminoacetamido. In some embodiments, R⁵ is 3-aminopropionamido. In some embodiments, R⁵ is pyroglutamylamino. In some embodiments, Q⁵ is selected from a group consisting of hydrogen, benzyl, amino, methylamino, hydrazino, trimethylammonium, pyrrolyl, acetamido, 2-aminoacetamido, 3-aminopropionamido, and pyroglutamylamino. In some embodiments, Q⁵ is selected from a group consisting of hydrogen, amino, hydrazino, trimethylammonium, pyrrolyl, acetamido, 2-aminoacetamido, 3-aminopropionamido, and pyroglutamylamino. In some embodiments, Q⁵ is selected from a group consisting of hydrogen, benzyl, amino, methylamino, hydrazino, pyrrolyl, and acetamido. In some embodiments, Q⁵ is hydrogen. In some embodiments, Q⁵ is benzyl. In some embodiments, Q⁵ is amino. In some embodiments, Q⁵ is methylamino. In some embodiments, Q⁵ is hydrazino. In some embodiments, Q⁵ is trimethylammonium. In some embodiments, Q⁵ is pyrrolyl. In some embodiments, Q⁵ is amino further linked with a substituent derived from a C-terminal carbon atom of an amino acid or peptide. In some embodiments, Q⁵ is acetamido. In some embodiments, Q⁵ is 2-aminoacetamido. In some embodiments, Q⁵ is 3-aminopropionamido. In some embodiments, Q⁵ is pyroglutamylamino.

In some embodiments, R⁶ is selected from a group consisting of hydroxyl, deprotonated hydroxyl, methoxyl, methylamino, ethylamino, and a substituent derived from an N-terminal nitrogen atom of an amino acid or peptide. In some embodiments, Q⁶ is selected from a group consisting of hydroxyl, deprotonated hydroxyl, methoxyl, ethoxyl, methylamino, ethylamino, and a substituent derived from an N-terminal nitrogen atom of an amino acid or peptide. The substituent derived from an N-terminal nitrogen atom of an amino acid or peptide may be represented as"-N_{AA}H-Z", where, N_{AA} is the N-terminal nitrogen atom of the amino acid or peptide, and Z is the remaining moiety of the amino acid or peptide. That is, Z-N_{AA}H₂ is an intact amino acid or peptide, particularly an amino acid, dipeptide or tripeptide. In some embodiments, the substituent derived from an N-terminal nitrogen atom of an amino acid or peptide is a substituent derived from an N-terminal nitrogen atom of an amino acid, dipeptide or tripeptide. In some embodiments, R⁶ is selected from a group consisting of hydroxyl, deprotonated hydroxyl, methoxyl, ethoxyl, methylamino, ethylamino, and a substituent derived from an N-terminal nitrogen atom of an amino acid, dipeptide or tripeptide. In some embodiments, Q⁶ is selected from a group consisting of hydroxyl, deprotonated hydroxyl, methoxyl, ethoxyl, methylamino, ethylamino, and a substituent derived from an N-terminal nitrogen atom of an amino acid, dipeptide or tripeptide. In some embodiments, the substituent derived from an N-terminal nitrogen atom of an amino acid or peptide is selected from a group consisting of *N*²-lysinyl (that is, a substituent derived from *N*² of L-lysine) and glycinyl prolinyl (that is, a substituent derived from an N-terminal nitrogen atom of L-prolyl-L-glycine). In some embodiments, R⁶ is selected from a group consisting of hydroxyl, deprotonated hydroxyl, methoxyl, ethoxyl, methylamino, ethylamino, *N*²-lysinyl, and glycinyl prolinyl. In some embodiments, R⁶ is selected from a group consisting of hydroxyl, methoxyl, methylamino, and ethylamino. In some embodiments, R⁶ is hydroxyl. In some embodiments, R⁶ is deprotonated hydroxyl. In some embodiments, R⁶ is methoxyl. In some embodiments, R⁶ is ethoxyl. In some embodiments, R⁶ is methylamino. In some embodiments, R⁶ is ethylamino. In some embodiments, R⁶ is a substituent derived from a N-terminal nitrogen atom of an amino acid or peptide. In some embodiments, R⁶ is *N*²-lysinyl. In some embodiments, R⁶ is glycinyl prolinyl. In some embodiments, Q⁶ is selected from a group consisting of hydroxyl, deprotonated hydroxyl, methoxyl, ethoxyl, methylamino, ethylamino, *N*²-lysinyl, and glycinyl prolinyl. In some embodiments, Q⁶ is selected from a group consisting of hydroxyl, methoxyl, methylamino, and ethylamino. In some embodiments, Q⁶ is hydroxyl. In some embodiments, Q⁶ is deprotonated hydroxyl. In some embodiments, Q⁶ is methoxyl. In some embodiments, Q⁶ is ethoxyl. In some embodiments, Q⁶ is methylamino. In some embodiments, Q⁶ is ethylamino. In some embodiments, Q⁶ is a substituent derived from a N-terminal nitrogen atom of an amino acid or peptide. In some embodiments, Q⁶ is *N*²-lysinyl. In some embodiments, Q⁶ is glycinyl prolinyl.

In some embodiments, *m* and *n* are each independently selected from a group consisting of 0 and 1. In some embodiments, *m* is 1. In some embodiments, *m* is 0. In some embodiments, *n* is 1. In some embodiments, *n* is 0. In some embodiments, *m* is 1, and *n* is 1. In some embodiments, *m* is 1, and *n* is 0. In some embodiments, *m* is 0, and *n* is 1. In some embodiments, *m* is 0, and *n* is 0.

In some embodiments, R¹ is the same as Q¹. In some embodiments, R² is the same as Q². In some embodiments, R³ is the same as Q³. In some embodiments, R⁴ is the same as Q⁴. In some embodiments, R⁵ is the same as Q⁵. In some embodiments, R⁶ is the same as Q⁶. In some embodiments, R¹ is different from Q¹. In some embodiments, R² is different from Q². In some embodiments, R³ is different from Q³. In some embodiments, R⁴ is different from Q⁴. In some embodiments, R⁵ is different from Q⁵. In some embodiments, R⁶ is different from Q⁶.

In some embodiments, R⁴ is hydrogen or methyl; R⁵ is selected from a group consisting of hydrogen, benzyl, amino, methylamino, hydrazino, pyrrolyl, and acetamido; R⁶ is selected from a group consisting of hydroxyl, methoxyl, methylamino, and ethylamino; and *n* is 1. In some embodiments, Q⁴ is hydrogen or methyl; Q⁵ is selected from a group consisting of hydrogen, benzyl, amino, methylamino, hydrazino, pyrrolyl, and acetamido; Q⁶ is selected from a group consisting of hydroxyl, methoxyl, methylamino, and ethylamino; and *m* is 1. In some embodiments, R⁴ is hydrogen; R⁵ is amino; R⁷ is -C(=O)-R⁶; and R⁶ is hydroxyl. In some embodiments, R⁴ is hydrogen; R⁵ is amino; R⁷ is -C(=O)-R⁶; and R⁶ is hydroxyl. In some embodiments, R⁴ is hydrogen; R⁵ is 3-aminopropionamido; R⁷ is -C(=O)-R⁶; and R⁶ is hydroxyl. In some embodiments, R⁴ is hydrogen; R⁵ is amino; and R⁷ is hydrogen. In some embodiments, Q⁴ is hydrogen; Q⁵ is amino; and Q⁶ is hydroxyl. In some embodiments, R¹ is hydrogen or hydroxyl, R² is hydrogen, and R³ is hydrogen. In some embodiments, Q¹ is hydrogen or hydroxyl; Q² is hydrogen; and Q³ is hydrogen. In some embodiments, R⁷ is -C(=O)-R⁶, R² is the same as Q², R³ is the same as Q³, R⁴ is the same as Q⁴, R⁵ is the same as Q⁵, and the R⁶ is the same as Q⁶. In some embodiments, at least two of R⁴, R⁵, and R⁷ are hydrogen. In some embodiments, R⁴ is hydrogen, and R⁵ is hydrogen. In some embodiments, R⁵ is hydrogen, and R⁷ is hydrogen. In some embodiments, Q⁴ is hydrogen, and Q⁵ is hydrogen.

Any chemical structure that is disclosed and contained in the present application and that is specified by formula (A) and/or any selectable specific moiety R¹, R², R³, R⁴, R⁵, R⁶, R⁷, Q¹, Q², Q³, Q⁴, Q⁵ and/or Q⁶, a position to which Q¹ is linked, and the specific parameters *m* and *n* in other structural formulas shall be regarded as further disclosing and containing a stereoisomer of the specified chemical structure. Any chemical structure disclosed or contained in the present application, particularly in examples, shall be regarded as further disclosing or containing a stereoisomer of the chemical structure.

In the present application, if the stereochemistry of any one or more chiral centers is not specified, it shall be regarded as disclosing or containing possible stereoisomers of the chiral center or combinations or mixtures of the stereoisomers. For example, if the stereochemistry of a chiral center is not specified, it shall be regarded as respectively disclosing or containing a chemical structure of a chiral center in an (*R*)-configuration, a chemical structure of a chiral center in an (*S*)-configuration, and a combination of the chiral center in any ratio, comprising a racemic mixture. For example, if the stereochemistry of two chiral centers is not specified, it shall be regarded as respectively disclosing or containing a chemical structure of the two chiral centers in an (*R*,*R*)-configuration, a chemical structure of the two chiral centers in an (*S*,*S*)-configuration, a chemical structure of the two chiral centers in an (*R*,*S*)-configuration, a chemical structure of the two chiral centers in an (*S*,*R*)-configuration, and a combination of the two chiral centers in any ratio.

In some embodiments, R⁴, R⁵, and R⁷ are genes which are different from each other. For example, R⁴ and R⁵ are different groups, and R⁷ is selected from a group consisting of hydroxymethyl and -C(=O)-R⁶, particularly -C(=O)-R⁶. In this case, a carbon to which R⁴, R⁵, and R⁷ are linked is an asymmetric carbon (hereinafter referred to as a "first asymmetric carbon").

As a specific example of the foregoing definition, any disclosed or contained chemical structure comprising the first asymmetric carbon shall be regarded, in a case that a configuration of the first asymmetric carbon is not further indicated, as simultaneously and respectively disclosing or containing two possible configurations of the first asymmetric carbon. Specifically, any disclosed or contained moiety of formula (A1) shall be regarded, in a case that the configuration of the first asymmetric carbon is not further indicated, as simultaneously and respectively disclosing or containing a moiety of formula (A1-R) and a moiety of formula (A1-S). In some embodiments, in formula (A) or formula (A1), a bond between the first asymmetric carbon and R⁴ is a bond of R⁴ that is in front of the plane of the paper, and a bond between the first asymmetric carbon and R⁵ is a bond of R⁵ that is behind the plane of the paper. That is, this is a moiety of formula (A1-R) in which the first asymmetric carbon is represented by "*". In a case that an atom, linked to the first asymmetric carbon, of R⁵ is a nitrogen atom, and R⁷ is selected from a group consisting of hydroxymethyl and -C(=O)-R⁶, the first asymmetric carbon is generally in the *R*-configuration. D-histidine has this configuration. In some embodiments, in formula (A) or formula (A1), a bond between the first asymmetric carbon and R⁴ is a bond of R⁴ that is in front of the plane of the paper, and a bond between the first asymmetric carbon and R⁵ is a bond of R⁵ that is behind the plane of the paper. That is, this is a moiety of formula (A1-S) in which the first asymmetric carbon is represented by "*". In a case that an atom, linked to the first asymmetric carbon, of R⁵ is a nitrogen atom, and R⁷ is selected from a group consisting of hydroxymethyl and -C(=O)-R⁶, the first asymmetric carbon is generally in the S-configuration. L-histidine has this configuration.

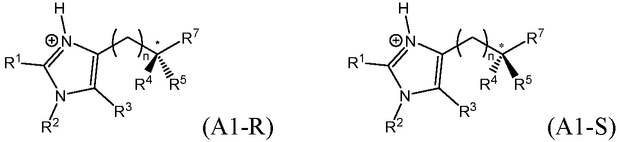

In some embodiments, Q⁴ and Q⁵ are groups which are different from each other. In this case, a carbon to which Q⁴ and Q⁵ are linked is an asymmetric carbon (hereinafter referred to as a second asymmetric carbon).

As a specific example of the foregoing definition, any disclosed or contained chemical structure comprising the second asymmetric carbon shall be regarded, in a case that a configuration of the second asymmetric carbon is not further indicated, as simultaneously and respectively disclosing or containing two possible configurations of the second asymmetric carbon. For example, any disclosed or contained moiety of formula (A2) shall be regarded, in a case that the configuration of the second asymmetric carbon is not further indicated, as simultaneously and respectively disclosing or containing a moiety of formula (A2-R) and a moiety of formula (A2-S). In some embodiments, in formula (A) or formula (A2), a bond between the second asymmetric carbon and Q⁴ is a bond of Q⁴ that is in front of the plane of the paper, and a bond between the second asymmetric carbon and Q⁵ is a bond of Q⁵ that is behind the plane of the paper. That is, this is a moiety of formula (A2-R) in which the second asymmetric carbon is represented by "*". In a case that an atom, linked to the second asymmetric carbon, of Q⁵ is a nitrogen atom, the second asymmetric carbon is generally in the *R*-configuration. D-histidine has this configuration. In some embodiments, in formula (A) or formula (A2), a bond between the second asymmetric carbon and Q⁴ is a bond of Q⁴ that is in front of the plane of the paper, and a bond between the second asymmetric carbon and Q⁵ is a bond of Q⁵ that is behind the plane of the paper. That is, this is a moiety of formula (A2-S) in which the second asymmetric carbon is represented by "*". In a case that an atom, linked to the second asymmetric carbon, of Q⁵ is a nitrogen atom, the second asymmetric carbon is generally in the *S*-configuration. L-histidine has this configuration.

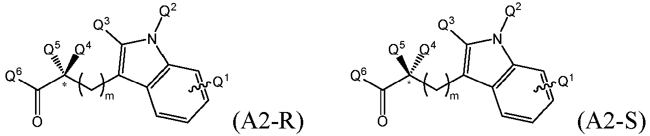

In some embodiments, the first asymmetric carbon is in the (*R*)-configuration. In some embodiments, the first asymmetric carbon is in the (*S*)-configuration. In some embodiments, the second asymmetric carbon is in the (*R*)-configuration. In some embodiments, the second asymmetric carbon is in the (*S*)-configuration. In some embodiments, the first asymmetric carbon is in the (*R*)-configuration, and the second asymmetric carbon is in the (*R*)-configuration. In some embodiments, the first asymmetric carbon is in the (*R*)-configuration, and the second asymmetric carbon is in the (*S*)-configuration. In some embodiments, the first asymmetric carbon is in the (*S*)-configuration, and the second asymmetric carbon is in the (*R*)-configuration. In some embodiments, the first asymmetric carbon is in the (*S*)-configuration, and the second asymmetric carbon is in the (*S*)-configuration.

In some embodiments, in formula (A), a bond between the first asymmetric carbon and R⁴ is a bond of R⁴ that is in front of the plane of the paper, a bond between the first asymmetric carbon and R⁵ is a bond of R⁵ that is behind the plane of the paper, a bond between the second asymmetric carbon and Q⁴ is a bond of Q⁴ that is in front of the plane of the paper, and a bond between the second asymmetric carbon and Q⁵ is a bond of Q⁵ that is behind the plane of the paper. In some embodiments, in formula (A), a bond between the first asymmetric carbon and R⁴ is a bond of R⁴ that is in front of the plane of the paper, a bond between the first asymmetric carbon and R⁵ is a bond of R⁵ that is behind the plane of the paper, a bond between the second asymmetric carbon and Q⁴ is a bond of Q⁴ that is behind the plane of the paper, and a bond between the second asymmetric carbon and Q⁵ is a bond of Q⁵ that is in front of the plane of the paper. In some embodiments, in formula (A), a bond between the first asymmetric carbon and R⁴ is a bond of R⁴ that is behind the plane of the paper, a bond between the first asymmetric carbon and R⁵ is a bond of R⁵ that is in front of the plane of the paper, a bond between the second asymmetric carbon and Q⁴ is a bond of Q⁴ that is in front of the plane of the paper, and a bond between the second asymmetric carbon and Q⁵ is a bond of Q⁵ that is behind the plane of the paper. In some embodiments, in formula (A), a bond between the first asymmetric carbon and R⁴ is a bond of R⁴ that is behind the plane of the paper, a bond between the first asymmetric carbon and R⁵ is a bond of R⁵ that is in front of the plane of the paper, a bond between the second asymmetric carbon and Q⁴ is a bond of Q⁴ that is behind the plane of the paper, and a bond between the second asymmetric carbon and Q⁵ is a bond of Q⁵ that is in front of the plane of the paper.

In some embodiments, R⁴ and R⁵ are groups which are different from each other, R⁷ is selected from a group consisting of hydroxymethyl and -C(=O)-R⁶, and Q⁴ and Q⁵ are groups which are different from each other. In some embodiments, R⁴ and R⁵ are groups which are different from each other, R⁷ is selected from a group consisting of hydroxymethyl and -C(=O)-R⁶, Q⁴ and Q⁵ are groups which are different from each other, the first asymmetric carbon is in the (*R*)-configuration, and the second asymmetric carbon is in the (*R*)-configuration, which is of formula (A-RR). In some embodiments, R⁴ and R⁵ are groups which are different from each other, R⁷ is selected from a group consisting of hydroxymethyl and -C(=O)-R⁶, Q⁴ and Q⁵ are groups which are different from each other, the first asymmetric carbon is in the (*R*)-configuration, and the second asymmetric carbon is in the (*S*)-configuration, which is of formula (A-RS). In some embodiments, R⁴ and R⁵ are groups which are different from each other, R⁷ is selected from a group consisting of hydroxymethyl and -C(=O)-R⁶, Q⁴ and Q⁵ are groups which are different from each other, the first asymmetric carbon is in the (*S*)-configuration, and the second asymmetric carbon is in the (*R*)-configuration, which is of formula (A-SR). In some embodiments, R⁴ and R⁵ are groups which are different from each other, R⁷ is selected from a group consisting of hydroxymethyl and -C(=O)-R⁶, Q⁴ and Q⁵ are groups which are different from each other, the first asymmetric carbon is in the (*S*)-configuration, and the second asymmetric carbon is in the (*S*)-configuration, which is of formula (A-SS).

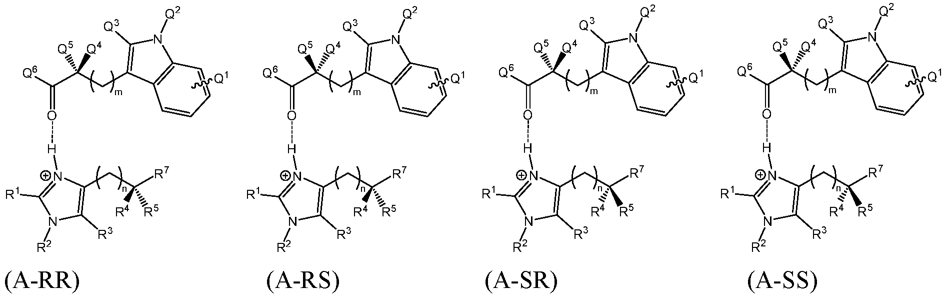

Any chemical structure that is disclosed and contained in the present application and that is specified by formula (A) and/or any selectable specific moiety R¹, R², R³, R⁴, R⁵, R⁶, R⁷, Q¹, Q², Q³, Q⁴, Q⁵ and/or Q⁶, a position to which Q¹ is linked, and the specific parameters *m* and *n* in other structural formulas shall be regarded as further disclosing and containing a chemically allowed tautomer of the specified chemical structure. Any chemical structure disclosed or contained in the present application, particularly in examples, shall be regarded as further disclosing or containing a chemically allowed tautomer, such as a keto-enol tautomer, of the chemical structure.

As a specific example of the foregoing definition, any disclosed or contained chemical structure "comprising a moiety with a double bond or carbon on an aromatic ring and hydroxyl or sulfhydryl on carbon" shall be regarded as simultaneously and further disclosing or containing a corresponding tautomer, that is, a chemical structure comprising a "carbonyl or thiocarbonyl" moiety. For example, any disclosed or contained chemical structure comprising a 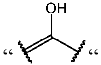 moiety shall be regarded as further disclosing or containing a corresponding tautomer comprising a 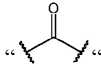 moiety, and any disclosed or contained chemical structure comprising a 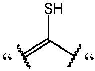 moiety shall be regarded as further disclosing or containing a corresponding tautomer comprising a 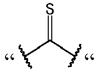 moiety.

As a specific example of the foregoing definition, any disclosed or contained chemical structure comprising a 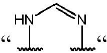 moiety in an aromatic ring shall be regarded as simultaneously and further disclosing or containing a corresponding tautomer comprising a 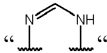 moiety. For example, any disclosed or contained chemical structure comprising a 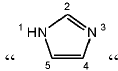 moiety in an imidazole ring shall be regarded as further disclosing or containing a corresponding tautomer comprising a " 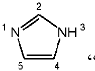 moiety.

Any resonance structure constituting a hybrid that is disclosed or contained in the present application shall be regarded as further disclosing or containing all other resonance structures constituting the hybrid and the hybrid itself.

As a specific example of the foregoing definition, any disclosed or contained chemical structure comprising a 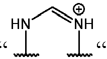 moiety in an aromatic ring shall be regarded as simultaneously and further disclosing or containing a corresponding resonance chemical structure comprising a 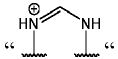 moiety. For example, any disclosed or contained chemical structure comprising a 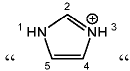 moiety in an imidazole ring shall be regarded as further disclosing or containing a resonance chemical structure comprising a 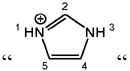 moiety, and more further disclosing or containing a corresponding hybrid (for example, represented as

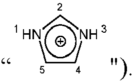

Any chemical structure that is disclosed or contained in the present application and that comprises a non-covalent bond, such as a hydrogen bond, that is formed by hydrogen on *N*¹ in 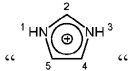 in a protonated imidazole ring shall be regarded as simultaneously and further disclosing or containing a corresponding chemical structure comprising a non-covalent bond, such as a hydrogen bond, that is formed by hydrogen on *N*³ in 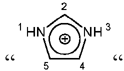 in a protonated imidazole ring. Any chemical structure that is disclosed or contained in the present application and that comprises a non-covalent bond, such as a hydrogen bond, that is formed by hydrogen on *N*^{τ} in protonated histidine, protonated histamine, protonated sarcosine or a similar compound with 1*H-*imidazol-4-yl or a group derived from 1*H*-imidazol-4-yl shall be regarded as simultaneously and further disclosing or containing a corresponding chemical structure comprising a non-covalent bond, such as a hydrogen bond, that is formed by hydrogen on *N*^{π} in protonated histidine, protonated histamine, protonated sarcosine or a similar compound with 1*H*-imidazol-4-yl or a group derived from 1*H*-imidazol-4-yl.

In addition to a disclosed or contained chemical structure in which a non-covalent bond is formed by hydrogen (*N*^{π}-H) that forms a non-covalent bond as of formula (A), any chemical structure that is disclosed and contained in the present application and in which R² is hydrogen shall be regarded as simultaneously and further disclosing or containing a corresponding chemical structure in which a non-covalent bond is formed by hydrogen (*N*^{τ}-H) on R², and vice versa. It should be understood that a structure of formula (A) in which R² is hydrogen and its corresponding structure of formula (A') may be tautomers. Any structure of formula (A) in which R² is hydrogen that is disclosed or contained in the present application shall be regarded as simultaneously and further disclosing or containing a structure of formula (A'), and vice versa.

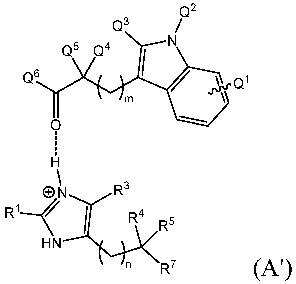

Similar to the structure of formula (A), correspondingly, the structure of formula (A') also comprises the first asymmetric carbon and the second asymmetric carbon.

In some embodiments, the structure of formula (A') is of formula (A'-RR). In some embodiments, the structure of formula (A') is of formula (A'-RS). In some embodiments, the structure of formula (A') is of formula (A'-SR). In some embodiments, the structure of formula (A') is of formula (A'-SS).

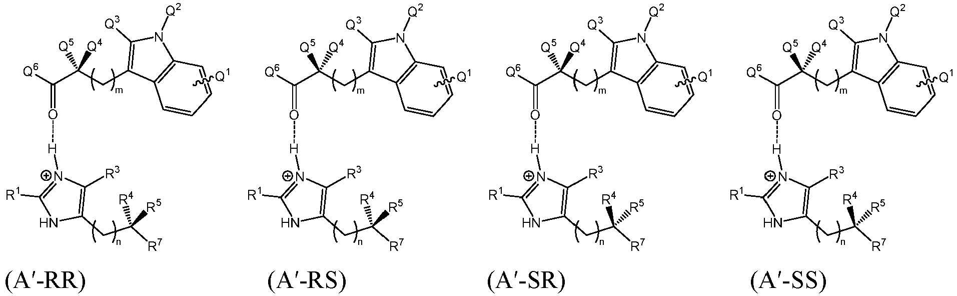

Any (Brønsted-Lowry) conjugate acid or base disclosed or contained in the present application shall be regarded as further disclosing or containing its corresponding or dominant conjugate acid-base pair under certain pH conditions that is chemically reasonable and that does not affect the main chemical structure and technical effects of the non-covalent dimer cation or salt of the present application. For example, if it is chemically reasonable and does not affect the main chemical structure and technical effects of the non-covalent dimer cation or salt of the present application, any disclosed or contained specific moiety or group that is a hydroxyl shall be regarded as further disclosing or containing a specific moiety or group that is deprotonated hydroxyl (-O⁻) or protonated hydroxyl (-OH₂⁺).

### Salt of the present application

In an aspect, the present application provides a salt.

In some embodiments, the salt of the present application comprises the non-covalent dimer cation of the present application, which particularly comprises the non-covalent dimer cation of the present application and a first anion. For example, the salt of the present application may be represented by a structure of formula (B) or a structure of formula (B'), where, X is the first anion.

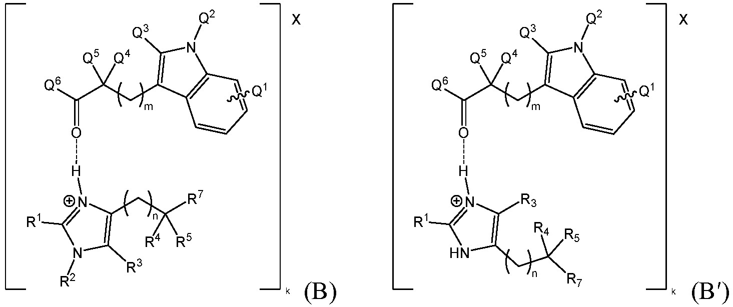

Through the first anion of the present application, the cation of the present application is enabled to exist stably in the form of a salt.

In some embodiments, the first anion (X in formula (B)) comprises or consists of at least one selected from a group consisting of a fluoride ion, a chloride ion, a bromide ion, an iodide ion, a sulfide ion, a nitrate ion, a sulfate ion, a sulfite ion, a thiosulfate ion, a persulfate ion, a selenate ion, a phosphate ion, a carbonate ion, a hexafluorophosphate ion, a hexafluorosilicate ion, an acetate ion, a sulfonate ion, a benzoate ion, and a polyphosphate ion. In some embodiments, the first anion comprises at least one selected from a group consisting of a hexafluorophosphate ion, a hexafluorosilicate ion, a chloride ion, and a sulfate ion. In some embodiments, the first anion is a fluoride ion. In some embodiments, the first anion is a chloride ion. In some embodiments, the first anion is a bromide ion. In some embodiments, the first anion is an iodide ion. In some embodiments, the first anion is a sulfide ion. In some embodiments, the first anion is a nitrate ion. In some embodiments, the first anion is a sulfate ion. In some embodiments, the first anion is a sulfite ion. In some embodiments, the first anion is a thiosulfate ion. In some embodiments, the first anion is a persulfate ion. In some embodiments, the first anion is a selenate ion. In some embodiments, the first anion is a phosphate ion. In some embodiments, the first anion is a carbonate ion. In some embodiments, the first anion is a hexafluorophosphate ion. In some embodiments, the first anion is a hexafluorosilicate ion. In some embodiments, the first anion is an acetate ion. In some embodiments, the first anion is a sulfonate ion such as a sulfamate ion. In some embodiments, the first anion is a benzoate ion. In some embodiments, the first anion is a polyphosphate ion.

A value of a parameter *k* is obtained based on the valence of the non-covalent dimer cation of the present application and the valence of the first anion. In a case of the non-covalent dimer cation of the present application with a monovalent positive electron, the value of the parameter *k* is equal to the valence of the first anion. In some embodiments, *k* is 1. In some embodiments, *k* is 2. In some embodiments, *k* is 3.

### Method for preparing the non-covalent dimer cation of the present application or the salt of the present application

In some embodiments, a method for preparing a salt of the non-covalent dimer cation of the present application, that is, the salt of the present application, is provided, which may comprise the following steps.

### Salt formation of the first reactant

A first reactant is contacted with a first acid. Then, a first acid salt of the first reactant is obtained. In some embodiments, the first acid salt of the first reactant comprises or consists of the first reactant and the first acid, and/or a conjugate base (a conjugate base of the first acid) of a protonated first reactant and the first acid,.

In some embodiments, the first reactant comprises, essentially consists of, in particular consists of, a compound of formula (C). In some embodiments, the first acid salt of the first reactant comprises, essentially consists of, in particular consists of, a salt of formula (C1). A is a conjugate base of the first acid, *a* is the valence of the conjugate base of the first acid, and *p* is the number of equivalents of H*ₐ*A. R¹, R², R³, R⁴, R⁵, R⁶, R⁷, and *n,* in formula (C) or formula (C1), may be the same as corresponding moieties or parameters of a desired cation (formula (A)) or salt (formula (B)) according to examples of the present application.

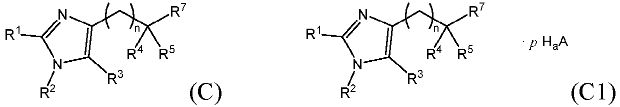

In some embodiments, the first reactant is contacted with the first acid in the presence of a first solvent, and particularly, in the first solvent. In some embodiments, the first acid is at least one selected from a group consisting of nitric acid, phosphoric acid, sulfuric acid, hydrofluoric acid, hydrochloric acid, hydrobromic acid, and hydroiodic acid, particularly hydrochloric acid. That is, the conjugate base (A in formula (C1)) of the first acid is at least one selected from a group consisting of a nitrate ion, a phosphate ion, a hydrogen phosphate ion, a dihydrogen phosphate ion, a sulfate ion, a hydrogen sulfate ion, a fluoride ion, a chloride ion, a bromide ion, and an iodide ion, particularly a chloride ion. In some embodiments, the first solvent is water. In formula (C1), the parameters *p* and *a* are obtained based on the type of the first acid. For example, in some embodiments, the first acid is hydrochloric acid, and *p* is 1.

Unless otherwise further defined, the term "conjugate acid" herein is intended to comprise a Brønsted-Lowry conjugate acid of a compound to which the term is directed. Furthermore, it does not only comprise a first conjugate acid obtained in a case that the compound acquires a proton, but also comprise, if chemically allowed, an acid obtained in a case that the compound further acquires more protons, such as a second conjugate acid obtained in a case that the first conjugate further acquires a proton, and a third conjugate acid obtained in a case that the second conjugate acid much further acquires a proton, and so on. Unless otherwise further defined, the term "conjugate base" herein is intended to comprise a Bronsted-Lowry conjugate base of a compound to which the term is directed. Further, it does not only comprise a first conjugate base obtained in a case that the compound loses a proton, but also comprises, if chemically allowed, a base obtained in a case that the compound further loses more protons, such as a second conjugate base obtained in a case that the first conjugate base further loses a proton, and a third conjugate base obtained in a case that the second conjugate base much further loses a proton, and so on. For example, unless otherwise further defined, a conjugate base of sulfuric acid herein shall be regarded as comprising not only a hydrogen sulfate ion, but also a sulfate ion. For example, unless otherwise further defined, a conjugate base of phosphoric acid herein shall be regarded as comprising not only a hydrogen phosphate ion, but also a dihydrogen phosphate ion and a phosphate ion.

In some embodiments, the first acid is in excess relative to the first reactant. In some embodiments, a molar ratio of the first reactant to the first acid is 1:2 to 1: 10, particularly 1:2.5 to 1:8, more particularly 1:4 to 1:6, and more particularly about 1:5.

In some embodiments, a concentration of the first acid in the first solvent is more than 1 M. In some embodiments, the concentration of the first acid in the first solvent is 1 M to 5 M. In some embodiments, the concentration of the first acid in the first solvent is about 2 M.

In some embodiments, the first reactant is contacted with the first acid at a first temperature. In some embodiments, the first temperature is 0°C to 70°C. In some embodiments, the first temperature is 10°C to 40°C, more particularly 20°C to 30°C, and more particularly about 25°C. In some embodiments, the first temperature is 20°C to 50°C, more particularly 30°C to 40°C, and more particularly about 37°C. In some embodiments, the first temperature is 0°C to 20°C, more particularly 0°C to 10°C, and more particularly about 4°C. In some embodiments, the first temperature is 40°C to 70°C, more particularly 50°C to 60°C, and more particularly about 60°C.

**In** some embodiments, after the first reactant is contacted with the first acid, heating and recrystallization are performed to obtain the first acid salt of the first reactant. **In** some embodiments, the heating temperature is 70°C to 100°C, particularly 80°C to 90°C, and more particularly about 85°C.

### Protonation of the first acid salt of the first reactant

**In** a first acidic condition, the first acid salt of the first reactant is contacted with a first salt that comprises a first anion, in particular consists of a first cation and the first anion. Then, a first solution that comprises a protonated first acid salt cation of the first reactant and the first anion and that is of formula (C2) is obtained.

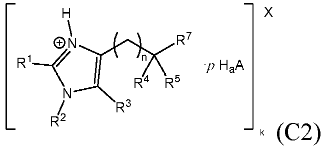

X and *k* in formula (C2) may be the same as corresponding moieties or parameters of a desired salt (formula (B)) according to an example of the present application.

In some embodiments, the first salt comprises or consists of at least one selected from a group consisting of ammonium fluoride, ammonium chloride, ammonium bromide, ammonium iodide, ammonium sulfide, ammonium nitrate, ammonium sulfate, ammonium sulfite, ammonium thiosulfate, ammonium persulfate, ammonium selenate, ammonium phosphate, ammonium carbonate, ammonium hexafluorophosphate, ammonium hexafluorosilicate, ammonium acetate, any ammonium sulfonate, ammonium benzoate, and ammonium polyphosphate. In some embodiments, the first salt comprises or consists of at least one selected from a group consisting of ammonium hexafluorophosphate, ammonium hexafluorosilicate, ammonium chloride, and ammonium sulfate. In some embodiments, the first salt is ammonium fluoride. In some embodiments, the first salt is ammonium chloride. In some embodiments, the first salt is ammonium bromide. In some embodiments, the first salt is ammonium iodide. In some embodiments, the first salt is ammonium sulfide. In some embodiments, the first salt is ammonium nitrate. In some embodiments, the first salt is ammonium sulfate. In some embodiments, the first salt is ammonium sulfite. In some embodiments, the first salt is ammonium thiosulfate. In some embodiments, the first salt is ammonium persulfate. In some embodiments, the first salt is ammonium selenate. In some embodiments, the first salt is ammonium phosphate. In some embodiments, the first salt is ammonium carbonate. In some embodiments, the first salt is ammonium hexafluorophosphate. In some embodiments, the first salt is ammonium hexafluorosilicate. In some embodiments, the first salt is ammonium acetate. In some embodiments, the first salt is any ammonium sulfonate such as ammonium sulfamate. In some embodiments, the first salt is ammonium benzoate. In some embodiments, the first salt is ammonium polyphosphate.

In some embodiments, the first acid salt of the first reactant is contacted with the first salt in the presence of a second solvent, and particularly, in the second solvent. In some embodiments, the second solvent is water. In some embodiments, the first cation is an ammonium ion. Here, the ammonium ion, as the first cation, may be taken as a hydrogen donor.

In some embodiments, the first salt is in excess relative to the first acid salt of the first reactant. In some embodiments, a molar ratio of the first acid salt of the first reactant to the first salt is 1:2 to 1:10, particularly 1:2.5 to 1:8, more particularly 1:4 to 1:6, and more particularly about 1:5.

In some embodiments, the first solution further comprises a conjugate base of the first acid. In some embodiments, the first solution further comprises the first cation. In some embodiments, the first solution further comprises the second solvent. In some embodiments, the first solution is essentially, and particularly, consisting of the protonated first acid salt cation of the first reactant, the first cation, the first anion, the conjugate base of the first acid, and the second solvent.

In some embodiments, the first acid salt of the first reactant is contacted with the first salt at a second temperature. In some embodiments, the second temperature is 0°C to 70°C. In some embodiments, the second temperature is 10°C to 40°C, more particularly 20°C to 30°C, and more particularly about 25°C. In some embodiments, the second temperature is 20°C to 50°C, more particularly 30°C to 40°C, and more particularly about 37°C. In some embodiments, the second temperature is 0°C to 20°C, more particularly 0°C to 10°C, and more particularly about 4°C. In some embodiments, the second temperature is 40°C to 70°C, more particularly 50°C to 60°C, and more particularly about 60°C. In some embodiments, the first temperature is the same as the second temperature.

In some embodiments, the pH of the first acidic condition is less than or equal to 6. In some embodiments, the pH of the first acidic condition is 4 to 6, particularly 4.5 to 5.5, particularly 4.8 to 5.2, particularly 4.9 to 5.1, and more particularly about 5. Generally, for the first anion that is less acidic or more basic, a first acidic condition with lower pH is selected. In some embodiments, the first anion is a hexafluorophosphate ion, and the pH of the first acidic condition is 4 to 6, particularly 4.5 to 5.5, particularly 4.8 to 5.2, particularly 4.9 to 5.1, and more particularly about 5.

In some embodiments, the first acidic condition is obtained by adding the first salt to the second solvent. In some embodiments, the first acidic condition is obtained by adding the first salt and an acid consisting of the first anion and a hydrogen ion to the second solvent.

### Formation of the non-covalent bond

In a second acidic condition, a second reactant is added to the first solution, and particularly, is contacted with the protonated first reactant cation in the first solution. Then, a bond is formed, and a second solution that comprises the cation (the first acid salt cation) according to the examples of the present application and the first anion and that is represented by a structure of formula (B1) or a structure of formula (B1') is obtained.

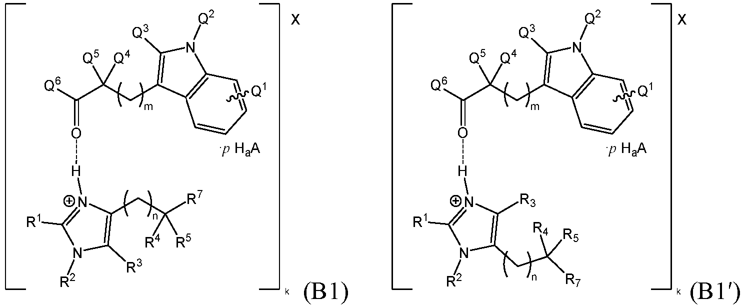

In some embodiments, the second reactant comprises, essentially consists of, in particular consists of, a compound of formula (D). Q¹, Q², Q³, Q⁴, Q⁵ Q⁶, a position to which Q¹ is linked, and *m,* may be the same as the desired cation (formula (A)) or salt (formula (B)) according to the examples of the present application.

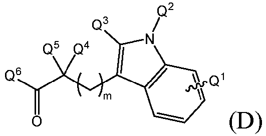

In some embodiments, the second reactant is in excess relative to the protonated first reactant cation. In some embodiments, a molar ratio of the protonated first reactant cation to the second reactant is 1:1 to 1:2, such as about 1:1.0, about 1:1.1, about 1:1.2, about 1:1.3, about 1:1.4, about 1:1.5, about 1:1.6, about 1:1.7, about 1:1.8, about 1:1.9, or about 1:2.0.

In some embodiments, the first solution further comprises a conjugate base of the first acid. In some embodiments, the first solution further comprises the first cation. In some embodiments, the first solution further comprises the second solvent. In some embodiments, the first solution is essentially, and particularly, consisting of the protonated first acid salt cation of the first reactant, the first cation, the first anion, the conjugate base of the first acid, and the second solvent.

In some embodiments, the second reactant is added to the first solution at a third temperature. In some embodiments, the third temperature is 0°C to 70°C. In some embodiments, the third temperature is 10°C to 40°C, more particularly 20°C to 30°C, and more particularly about 25°C. In some embodiments, the third temperature is 20°C to 50°C, more particularly 30°C to 40°C, and more particularly about 37°C. In some embodiments, the third temperature is 0°C to 20°C, more particularly 0°C to 10°C, and more particularly about 4°C. In some embodiments, the third temperature is 40°C to 70°C, more particularly 50°C to 60°C, and more particularly about 60°C. In some embodiments, the first temperature is the same as the third temperature. In some embodiments, the second temperature is the same as the third temperature. In some embodiments, the first temperature, the second temperature, and the third temperature are the same.

In some embodiments, the pH of the second acidic condition is less than or equal to 6. In some embodiments, the pH of the second acidic condition is 4 to 6, particularly 4.5 to 5.5, particularly 4.8 to 5.2, particularly 4.9 to 5.1, and more particularly about 5. Generally, for the first anion that is less acidic or more basic, a second acidic condition with lower pH is selected. In some embodiments, the first anion is a hexafluorophosphate ion, and the pH of the second acidic condition is 4 to 6, particularly 4.5 to 5.5, particularly 4.8 to 5.2, particularly 4.9 to 5.1, and more particularly about 5. In some embodiments, the pH of the first acidic condition is the same as that of the second acidic condition.

In some embodiments, the second acidic condition is obtained by adding the first salt to the second solvent. In some embodiments, the second acidic condition is obtained by adding the first salt and an acid consisting of the first anion and a hydrogen ion to the second solvent. In some embodiments, a reductant is further added to the first solution, and particularly, the reductant is added to the first solution before, after or at the same time as the second reactant is added to the first solution. That is, in some embodiments, the second acidic condition is reductive, and particularly strongly reductive. The non-covalent dimer cation of the present application has strong reducing capacity. Therefore, addition of the reductant is conductive to reduction of a proportion of the non-covalent dimer cation of the present application that is oxidized during reaction, and is conductive to increase of the yield of the salt of the present application. In some embodiments, the reductant comprises at least one or more selected from a group consisting of vitamin C, vitamin E, and a derivative of vitamin C or vitamin E.

In some embodiments, the second solution is further purified to obtain a first intermediate comprising the cation according to the examples of the present application, i.e., the first acid salt cation, and the first anion. In some embodiments, the purification comprises dialysis. In some embodiments, the dialysis may be performed through a membrane with molecular weight cut-off (MWCO) of 50 Da to 500 Da, in particular about 100 Da or 200 Da.

### Formation of the salt of the present application

A first base is added to the second solution or contacted with the first intermediate to remove the first acid (H*ₐ*A in formula (B1)) in the second solution or the first intermediate. Then, the salt of the present application is obtained.

In some embodiments, the salt of the present application comprises the non-covalent dimer cation of the present application and the first anion. For example, the salt of the present application may be of formula (B).

In some embodiments, the first base comprises or consists of, but is not limited to, at least one selected from a group consisting of ammonia water, sodium hydroxide, potassium hydroxide, and triethanolamine. Those skilled in the art should understand that any base that can adjust the pH and make a solution alkaline without causing a further redox reaction can be taken as the first base. In some embodiments, the first base is ammonia water, in particular diluted ammonia water. In some embodiments, the first base is sodium hydroxide. In some embodiments, the first base is potassium hydroxide. In some embodiments, the first base is triethanolamine.

In some embodiments, the first base is added to the second solution or contacted with the first intermediate to adjust the pH of the second solution or a solution comprising the first intermediate to be alkaline. In some embodiments, the alkalinity refers that the pH is higher than 8, particularly 8 to 10, particularly 8.5 to 9.5, particularly 8.8 to 9.2, and more particularly about 9.

In some embodiments, contacting the first intermediate with the first base refers that the first intermediate is contacted with the first base in the presence of water.

In some embodiments, after the first base is added to the second solution, purification is further performed to obtain the salt of the present application. In some embodiments, the purification comprises dialysis. In some embodiments, the dialysis may be performed through a membrane with molecular weight cut-off (MWCO) of 50 Da to 500 Da, in particular about 100 Da or 200 Da. In some embodiments, the purification comprises filtration.

### Use of the non-covalent dimer cation of the present application or the salt of the present application

In some embodiments, use of the cation or salt of the present application in promotion of cell repair is provided. In some embodiments, use of the cation or salt of the present application in preparation of a drug for promoting cell repair is provided. In some embodiments, a method for promoting cell repair is provided, comprising administering the cation or salt of the present application. In some embodiments, the cation or salt of the present application for use in promotion of cell repair is provided.

In some embodiments, use of the cation or salt of the present application in antioxidation is provided. In some embodiments, use of the cation or salt of the present application in preparation of an antioxidant is provided. In some embodiments, a method for antioxidation is provided, comprising administering the cation or salt of the present application. In some embodiments, the cation or salt of the present application for use in antioxidation provided. In some embodiments, the antioxidant is a drug for inhibiting, reducing or reversing oxidative stress in human or animal cells. In some embodiments, the antioxidant is a drug for inhibiting, reducing or scavenging reactive oxygen species in human or animal bodies.

In some embodiments, use of the cation or salt of the present application in inhibition, reduction or scavenging of reactive oxygen species in human or animal bodies is provided. In some embodiments, use of the cation or salt of the present application in preparation of a drug for inhibiting, reducing or scavenging reactive oxygen species in human or animal bodies is provided. In some embodiments, a method for inhibiting, reducing or scavenging reactive oxygen species in human or animal bodies is provided, comprising administering the cation or salt of the present application, in particular to the human or animal cells. In some embodiments, the cation or salt provided by the present application for use in inhibiting, reducing or scavenging reactive oxygen species in human or animal bodies.

In some embodiments, use of the cation or salt of the present application in treatment of a disease or a symptom related to reactive oxygen species is provided. In some embodiments, use of the cation or salt of the present application in preparation of a drug for treating a disease or a symptom related to reactive oxygen species is provided. In some embodiments, a method for treating a disease or a symptom related to reactive oxygen species is provided, comprising: administering the cation or salt of the present application, in particular to a patient with a disease or a symptom related to reactive oxygen species. In some embodiments, use of the cation or salt of the present application in treatment of a disease or a symptom related to reactive oxygen species is provided.

In some embodiments, the promotion of cell repair comprises, but is not limited to: the treatment of aging, inflammation, overweight, obesity, cancer, tumor, hepatopathy, neurodegenerative diseases (e.g., Parkinson's disease or Alzheimer's disease), arterial hypertension, atherosclerosis, cardiovascular disease, diabetes, hypercholesterolemia, chronic fatigue syndrome, ischemia reperfusion damage, neurodegenerative disease, ultraviolet-induced damage, or alcohol-induced damage. In some embodiments, the promotion of cell repair is the treatment of mucosal damage, in particular to gastric mucosal damage. In some embodiments, the promotion of cell repair is the treatment of alcohol-induced damage, in particular to alcohol-induced gastric mucosal damage.

In some embodiments, the disease or the symptom related to reactive oxygen species comprises but is not limited to: aging, inflammation, overweight, obesity, cancer, tumor, hepatopathy, neurodegenerative diseases (e.g., Parkinson's disease or Alzheimer's disease), arterial hypertension, atherosclerosis, cardiovascular disease, diabetes, hypercholesterolemia, chronic fatigue syndrome, ischemia reperfusion damage, neurodegenerative disease, ultraviolet-induced damage, or alcohol-induced damage. In some embodiments, the disease or the symptom related to reactive oxygen species is gastric mucosal damage. In some embodiments, the disease or the symptom related to reactive oxygen species is alcohol-induced damage, in particular to alcohol-induced gastric mucosal damage.

In some embodiments, the disease or the symptom related to reactive oxygen species is neurodegenerative disease. In some embodiments, the neurodegenerative disease is Parkinson's disease or Alzheimer's disease. In some embodiments, the neurodegenerative disease is Parkinson's disease. In some embodiments, the neurodegenerative disease is Alzheimer's disease. In some embodiments, the neurodegenerative disease comprises: increase of 2-phenyl-4,5-dihydro-4,4,5,5-tetramethyl-1H-imidazolyl-1-oxyl-3-oxide, or increase of 6-hydroxydopamine, and/or a disease related to increase of β-amyloid. In some embodiments, the neurodegenerative disease comprises: a disease related to increase of 2-phenyl-4,5-dihydro-4,4,5,5-tetramethyl-14-imidazolyl-1-oxyl-3-oxide. In some embodiments, the neurodegenerative disease comprises a disease related to increase of 6-hydroxydopamine. In some embodiments, the neurodegenerative disease is a disease related to increase of β-amyloid.

In some embodiments, the cation or salt of the present application can effectively act on a repair-promoting signaling pathway in a cell, enhance a self-repair function of the cell and make it have the ability for self-repair, thereby preventing, treating or alleviating diseases that are susceptible to, such as Parkinson's disease, Alzheimer's disease and other neurodegenerative diseases, due to the weakening of the ability of cell repair in terms of cell functions.

In some embodiments, use of the cation or salt of the present application in promotion of cell proliferation is provided. In some embodiments, use of the cation or salt of the present application in preparation of a drug for promoting cell proliferation is provided. In some embodiments, a method for promoting cell proliferation is provided, comprising: administering the cation or salt of the present application. In some embodiments, use of the cation or salt of the present application in promotion of cell proliferation is provided. In some embodiments, the cells are cells located on the skin. In some embodiments, the cells are skin cells. In some embodiments, the cells are selected from a group consisting of epidermal fibroblasts, keratinocytes, vascular endothelial cells, and nerve cells. In some embodiments, the cells are epidermal fibroblasts. In some embodiments, the cells are keratinocytes. In some embodiments, the cells are vascular endothelial cells. In some embodiments, the cells are nerve cells, in particular to nerve cells of the skin. In some embodiments, the cells belong to epithelial tissues. In some embodiments, the cells belong to connective tissues. In some embodiments, the cells belong to muscular tissues. In some embodiments, the cells belong to nervous tissues.

In some embodiments, use of the cation or use salt of the present application in the treatment, alleviation or repair of a wound is provided. In some embodiments, use of the cation or salt of the present application in preparation of a drug for treating, alleviating or repairing a wound is provided. In some embodiments, a method for treating, alleviating or repairing a wound is provided, comprising: administering the cation or salt of the present application. In some embodiments, use of the cation or salt of the present application in the treatment, alleviation or repair of a wound is provided. In some embodiments, a wound comprise a skin wound.

In some embodiments, the cation or salt of the present application can activate a signaling pathways that promotes cell proliferation in cells from living organisms, so that a variety of cells can accelerate cell proliferation activities under the premise of regulation, thereby promoting the healing and repair of a wound and improving an effect of wound repair. In some embodiments, the cation or salt of the present application can improve and treat diseases involving in insufficient cell proliferation, or accelerate a wound healing process.

### Examples

In the following examples, if a compound of an example comprises a first reactant, a second reactant, a cation of this example, or a salt of this example, and has chirality that is not explicitly specified, the compound may be a chiral mixture such as a racemic compound.

### Example 1

A salt of Example 1 was specifically shown in formula (1) or formula (1'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n =* 1*, m =* 1*, k =* 1, and X was a hexafluorophosphate ion.

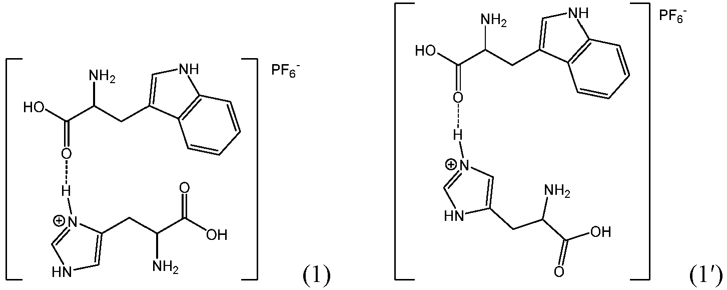

### Preparation method

A method for preparing the salt in this example was as follows, in which:
- a first reactant was 2-amino-3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The hydrochloride of the first reactant in this example was white crystals, with a yield of about 0.8 g and a productivity rate of about 80%.

The yield of the salt of this example was about 1.6 g, and the productivity rate was about 85%.

The salt of this example was detected by using a high-resolution mass spectrometer, to obtain the cation of the present application, (C₁₇H₂₂N₅O₄)⁺, m/z =360.4.

### Example 2

A salt of Example 2 was specifically shown in Formula (2) or Formula (2'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1*, m =* 1*, k =* 1, and X was a hexafluorophosphate ion.

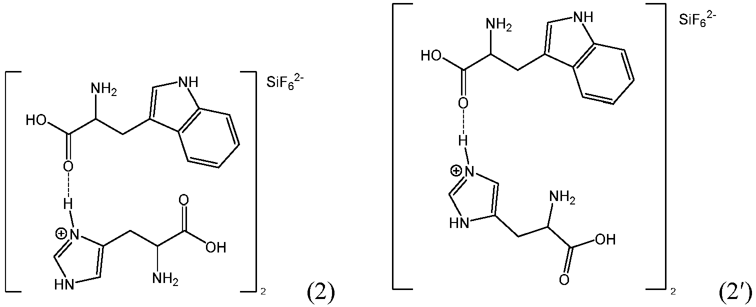

### Preparation method

A method for preparing the salt in this example was as follows, in which:
- a first reactant was 2-amino-3-(1*H*-imidazol-4-yl)propionic acid;
- a second reactant was 2-amino-3-(1*H*-imidazol-3-yl)propionic acid; and
- a first salt was ammonium hexafluorosilicate, i.e. a first anion was a hexafluorosilicate ion.

1 g of first reactant was placed into 10 mL of 2 M hydrochloric acid at 37°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 37°C for 12 h to obtain a first aqueous solution comprising a protonated hydrochloride cation of the first reactant and the first anion.

0.89 g of second reactant was put into the first aqueous solution at 37°C and stirred gently for 36 h, to obtain a second aqueous solution comprising the cation of this example. A 1 M sodium hydroxide solution was added to the second aqueous solution to adjust the pH to 9. The second aqueous solution was completely dialyzed with a dialysis bag with molecular weight cut-off of 200 Da. The dialyzed second aqueous solution was dried to obtain a product, that is, the salt of this example.

### Detection of the product or the intermediate

The hydrochloride of the first reactant in this example was white crystals, with a yield of about 0.8 g and a productivity rate of about 80%.

The yield of the salt of this example was about 1.2 g, and the productivity rate was about 63%.

The salt of this example was detected by using a high-resolution mass spectrometer, to obtain the cation of the present application, (C₁₇H₂₂N₅O₄)⁺, m/z =360.4.

### Example 3

A salt of Example 3 was specifically shown in Formula (3) or Formula (3'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n =* 1*, m =* 1*, k =* 1, and X was a chloride ion.

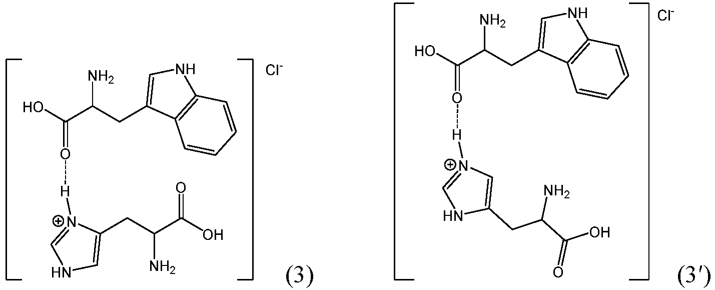

### Preparation method

A method for preparing the salt in this example was as follows, in which:
- a first reactant was 2-amino-3-(1H-imidazol-4-yl)propionic acid;
- a second reactant was 2-amino-3-(1H-imidazol-3-yl)propionic acid; and
- a first salt was ammonium chloride, i.e. a first anion was a chloride ion.

1 g of first reactant was placed into 10 mL of 2 M hydrochloric acid at 60°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 2 g of first salt of this example was added, and the mixture was stirred at 60°C for 6 h to obtain a first aqueous solution comprising a protonated hydrochloride cation of the first reactant and the first anion.

0.65 g of second reactant was put into the first aqueous solution at 60°C and stirred gently for 48 h, to obtain a second aqueous solution comprising the cation of this example. A 1 M sodium hydroxide solution was added to the second aqueous solution to adjust the pH to 9. The second aqueous solution was completely dialyzed with a dialysis bag with molecular weight cut-off of 200 Da. The dialyzed second aqueous solution was dried to obtain a product, that is, the salt of this example.

### Detection of the product or the intermediate

The hydrochloride of the first reactant in this example was white crystals, with a yield of about 0.8 g and a productivity rate of about 80%.

The yield of the salt of this example was about 0.6 g, and the productivity rate was about 32%.

The salt of this example was detected by using a high-resolution mass spectrometer, to obtain the cation of the present application, (C₁₇H₂₂N₅O₄)⁺, m/z =360.4.

### Example 4

A salt of Example 4 was specifically shown in Formula (4) or Formula (4'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydroxyl located in position 5 of an indole moiety, Q² was hydrogen, Q³ was hydrogen, Q⁴ was hydrogen, Q⁵ was amino, Q⁶ was hydroxyl, *n* = 1, *m* = 1, *k* = 1, and X was a hexafluorophosphate ion.

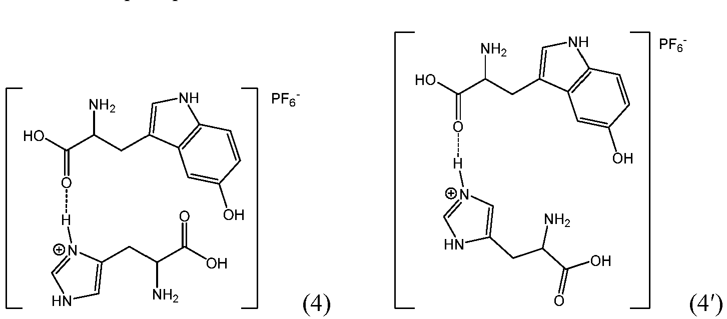

### Preparation method

A method for preparing the salt in this example was as follows, in which:
- a first reactant was 2-amino-3-(1H-imidazol-4-yl)propionic acid;
- a second reactant was 2-amino-3-(5-hydroxyl-1H-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 4°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 2 g of first salt of this example was added, and the mixture was stirred at 4°C for 6 h to obtain a first aqueous solution comprising a protonated hydrochloride cation of the first reactant and the first anion.

1.5 g of second reactant was put into the first aqueous solution at 4°C and stirred gently for 48 h, to obtain a second aqueous solution comprising the cation of this example. A 1 M sodium hydroxide solution was added to the second aqueous solution to adjust the pH to 9. The second aqueous solution was completely dialyzed with a dialysis bag with molecular weight cut-off of 200 Da. The dialyzed second aqueous solution was dried to obtain a product, that is, the salt of this example.

### Detection of the product or the intermediate

The hydrochloride of the first reactant in this example was white crystals, with a yield of about 0.8 g and a productivity rate of about 80%.

The yield of the salt of this example was about 1.6 g, and the productivity rate was about 64%.

The salt of this example was detected by using a high-resolution mass spectrometer, to obtain the cation of the present application, (C₁₇H₂₂N₅O₅)⁺, m/z =376.3.

### Example 5

A salt of Example 5 was specifically shown in Formula (5) or Formula (5'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydroxyl located in position 5 of an indole moiety, Q² was hydrogen, Q³ was hydrogen, Q⁴ was hydrogen, Q⁵ was amino, Q⁶ was hydroxyl, *n* = 1, *m* = 1, *k* = 2, and X was a sulfate ion.

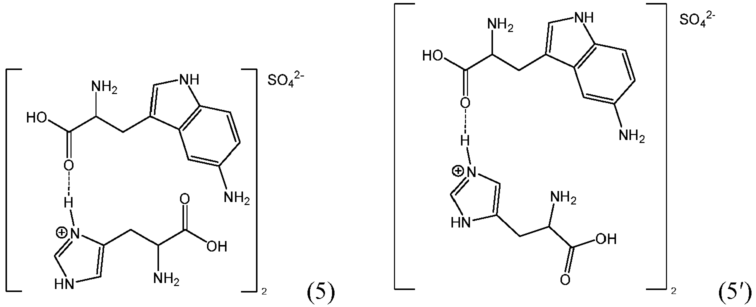

### Preparation method

A method for preparing the salt in this example was as follows, in which:
- a first reactant was 2-amino-3-(1H-imidazol-4-yl)propionic acid;
- a second reactant was 2-amino-3-(5-amino-1H-imidazol-3-yl)propionic acid; and
- a first salt was ammonium sulfate, i.e. a first anion was a sulfate ion.

1 g of first reactant was placed into 10 mL of 2 M hydrochloric acid at 4°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 3 g of first salt of this example was added, and the mixture was stirred at 4°C for 6 h to obtain a first aqueous solution comprising a protonated hydrochloride cation of the first reactant and the first anion.

1.5 g of second reactant was put into the first aqueous solution at 4°C and stirred gently for 48 h, to obtain a second aqueous solution comprising the cation of this example. A 1 M sodium hydroxide solution was added to the second aqueous solution to adjust the pH to 9. The second aqueous solution was completely dialyzed with a dialysis bag with molecular weight cut-off of 200 Da. The dialyzed second aqueous solution was dried to obtain a product, that is, the salt of this example.

### Detection of the product or the intermediate

The hydrochloride of the first reactant in this example was white crystals, with a yield of about 0.8 g and a productivity rate of about 80%.

The yield of the salt of this example was about 1.6 g, and the productivity rate was about 64%.

The salt of this example was detected by using a high-resolution mass spectrometer, to obtain the cation of the present application, (C₁₇H₂₃N₆O₄)⁺, m/z =375.2.

### Example 6

A salt of Example 6 was specifically shown in Formula (6) or Formula (6'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was methyl located in position 4 of an indole moiety, Q² was hydrogen, Q³ was hydrogen, Q⁴ was hydrogen, Q⁵ was amino, Q⁶ was hydroxyl, *n = 1, m = 1, k =* 1, and X was a hexafluorophosphate ion.

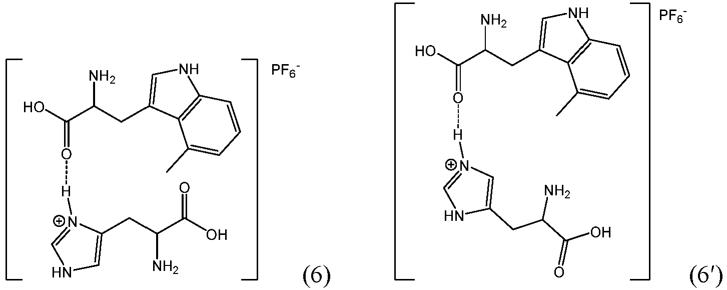

A method for preparing the salt in this example was as follows, in which:
- a first reactant was 2-amino-3-(1*H*-imidazol-4-yl)propionic acid;
- a second reactant was 2-amino-3-(4-amino-1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄N₅O₄)⁺, m/z =374.2.

### Example 7

A salt of Example 7 was specifically shown in formula (7) or formula (7'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was cyano located in position 5 of an indole moiety, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

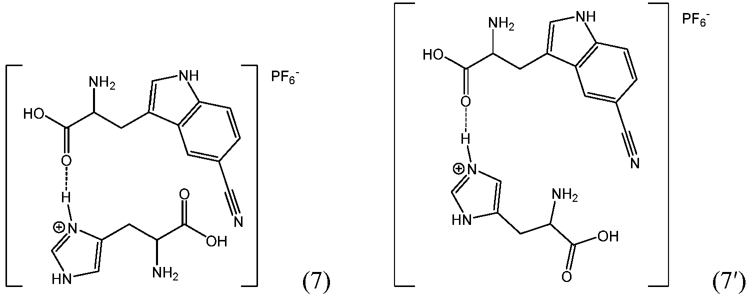

A method for preparing the salt in this example was as follows, in which:
- a first reactant was 2-amino-3-(1*H*-imidazol-4-yl)propionic acid;
- a second reactant was 2-amino-3-(5-cyano-1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₁N₆O₄)⁺, m/z =385.1.

### Example 8

A salt of Example 8 was specifically shown in formula (8) or formula (8'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was chloro located in position 5 of an indole moiety, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

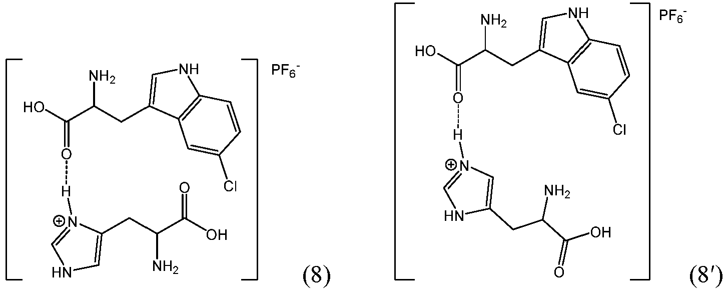

A method for preparing the salt in this example was as follows, in which:
- a first reactant was 2-amino-3-(1*H*-imidazol-4-yl)propionic acid;
- a second reactant was 2-amino-3-(5- chloro-1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₇H₂₁N₅O₄Cl)⁺, m/z =394.9.

### Example 9

A salt of Example 9 was specifically shown in formula (9) or formula (9'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was carboxyl located in position 6 of an indole moiety, Q² was hydrogen, Q³ was hydrogen, Q⁴ was hydrogen, Q⁵ was amino, Q⁶ was hydroxyl, *n* = 1, *m* = 1, *k* = 1, and X was a hexafluorophosphate ion.

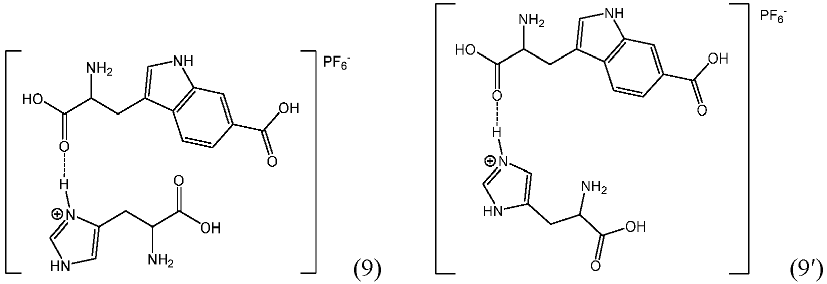

A method for preparing the salt in this example was as follows, in which:
- a first reactant was 2-amino-3-(1*H*-imidazol-4-yl)propionic acid;
- a second reactant was 2-amino-3-(6-carboxyl-1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₂N₅O₆)⁺, m/z =404.1.

### Example 10

A salt in Example 10 was specifically shown in formula (10) or formula (10'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was methyl, Q³ was hydrogen, Q⁴ was hydrogen, Q⁵ was amino, Q⁶ was hydroxyl, *n* = 1, *m* = 1, *k* = 1, and X was a hexafluorophosphate ion.

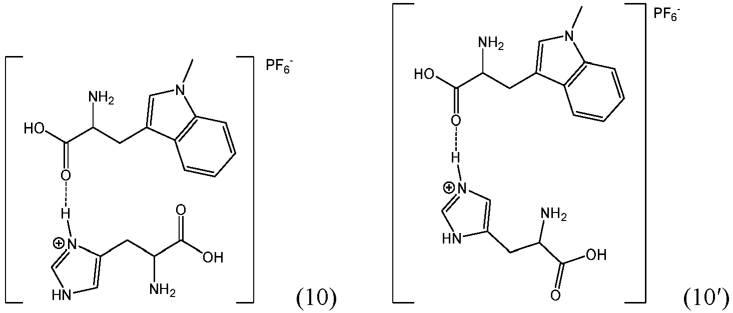

A method for preparing the salt in this example was as follows, in which:
- a first reactant was 2-amino-3-(1*H*-imidazol-4-yl)propionic acid;
- a second reactant was 2-amino-3-(1-methyl-1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄N₅O₄)⁺, m/z =374.2.

### Example 11

A salt of Example 11 was specifically shown in formula (11) or formula (11'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was methyl, Q⁵ was hydrogen, Q⁶ was hydroxyl, *n* = 1, *m* = 1, *k =* 1, and X was a hexafluorophosphate ion.

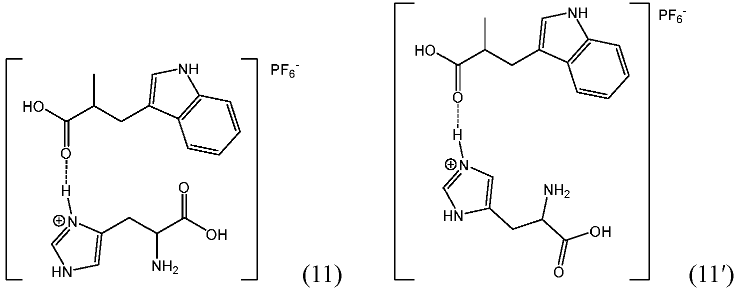

A method for preparing the salt in this example was as follows, in which:
- a first reactant was 2-amino-3-(1*H*-imidazol-4-yl)propionic acid;
- a second reactant was 2-methyl-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₃N₄O₄)⁺, m/z =359.3.

### Example 12

A salt of Example 12 was specifically shown in formula (12) or formula (12'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was methylamino, Q⁶ was hydroxyl, *n* = 1, *m* = 1, *k =* 1, and X was a hexafluorophosphate ion.

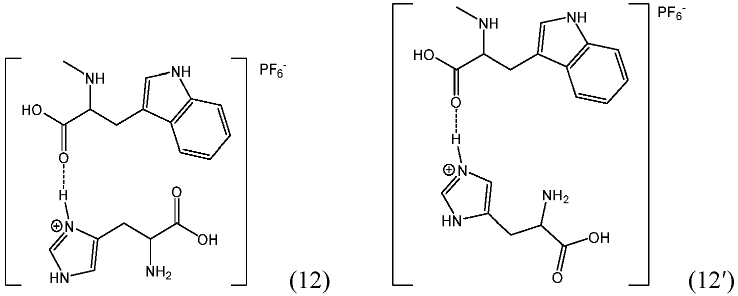

A method for preparing the salt in this example was as follows, in which:
- a first reactant was 2-amino-3-(1*H*-imidazol-4-yl)propionic acid;
- a second reactant was 2-methylamino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄N₅O₄)⁺, m/z =374.3.

### Example 13

A salt of Example 13 was specifically shown in formula (13) or formula (13'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was acetamido, Q⁶ was hydroxyl, *n* = 1, *m* = 1, *k =* 1, and X was a hexafluorophosphate ion.

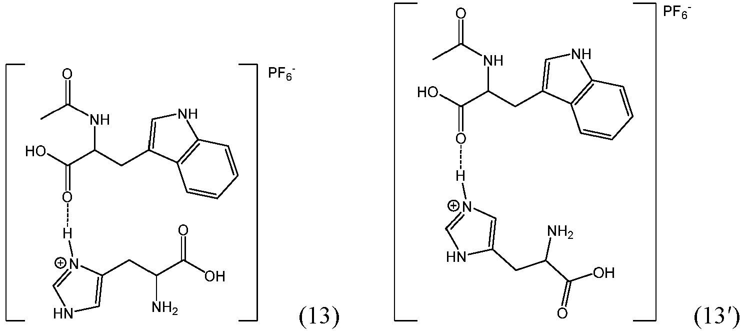

A method for preparing the salt in this example was as follows, in which:
- a first reactant was 2-amino-3-(1*H*-imidazol-4-yl)propionic acid;
- a second reactant was 2-acetamido-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₉H₂₄N₅O₅)⁺, m/z =402.2.

### Example 14

A salt of Example 14 was specifically shown in formula (14) or formula (14'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was pyrrolyl, Q⁶ was hydroxyl, *n* = 1, *m* = 1, *k =* 1, and X was a hexafluorophosphate ion.

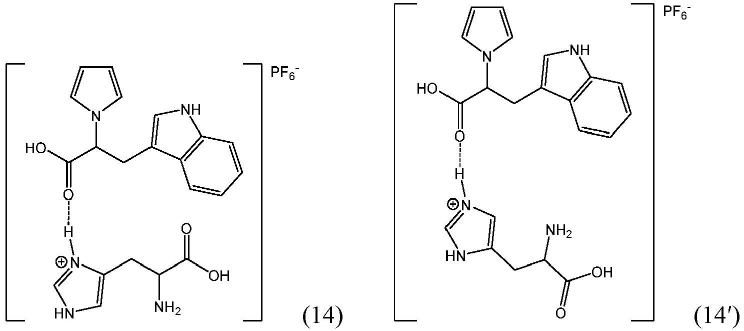

A method for preparing the salt in this example was as follows, in which:
- a first reactant was 2-amino-3-(1*H*-imidazol-4-yl)propionic acid;
- a second reactant was 2-(1H-pyrrol-1-yl)-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₂₁H₂₄N₅O₄)⁺, m/z =410.1.

### Example 15

A salt of Example 15 was specifically shown in formula (15) or formula (15'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was phenylmethyl, Q⁶ was hydroxyl, *n* = 1, *m* = 1, *k =* 1, and X was a hexafluorophosphate ion.

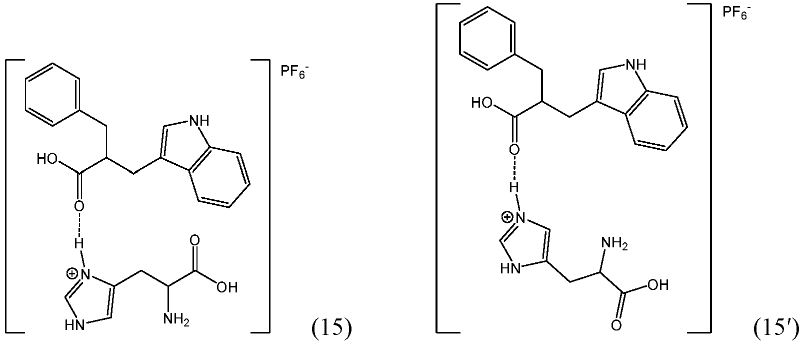

A method for preparing the salt in this example was as follows, in which:
- a first reactant was 2-amino-3-(1*H*-imidazol-4-yl)propionic acid;
- a second reactant was 2-phenylmethyl-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₂₄H₂₇N₄O₄)⁺, m/z =429.1.

### Example 16

A salt of Example 16 was specifically shown in formula (16) or formula (16'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was methoxyl, *n* = 1, *m* = 1, *k =* 1, and X was a hexafluorophosphate ion.

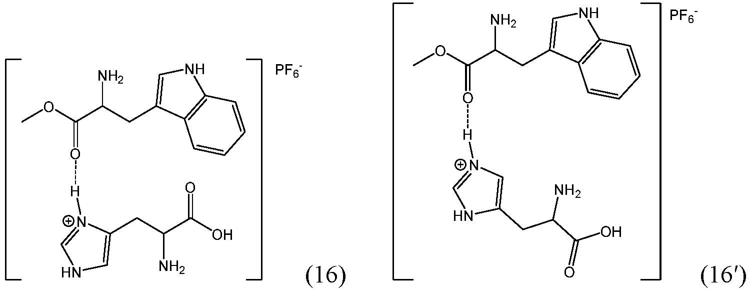

A method for preparing the salt in this example was as follows, in which:
- a first reactant was 2-amino-3-(1*H*-imidazol-4-yl)propionic acid;
- a second reactant was 2-amino-3-(1*H*-imidazol-3-yl)methylpropionate; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄N₅O₄)⁺, m/z =374.3.

### Example 17

A salt of Example 17 was specifically shown in formula (17) or formula (17'). That is, R¹ was hydrogen, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was ethylamino, *n* = 1, *m* = 1, *k =* 1, and X was a hexafluorophosphate ion.

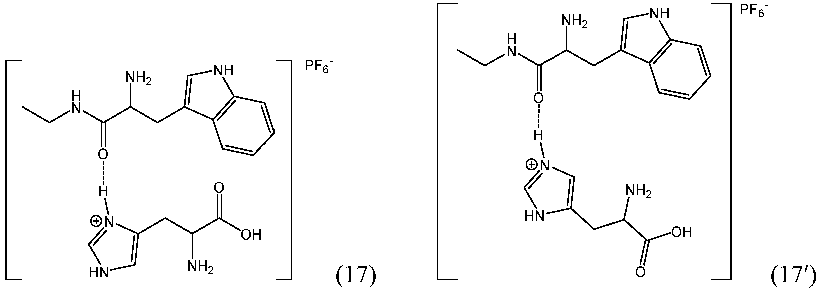

A method for preparing the salt in this example was as follows, in which:
- a first reactant was 2-amino-3-(1*H*-imidazol-4-yl)propionic acid;
- a second reactant is 2-amino-3-(1*H*-imidazol-3-yl)ethylpropionamide; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₉H₂₇O₃N₆)⁺, m/z =387.2.

### Example 18

A salt of Example 18 was specifically shown in formula (18) or formula (18'). That is, R¹ was hydroxyl, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

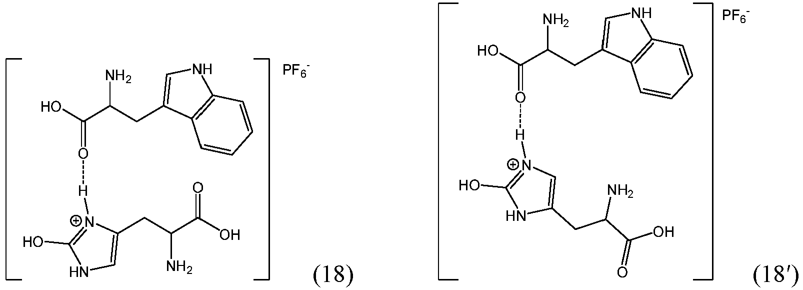

A method for preparing the salt in this example was as follows, in which:
- a first reactant was 2-amino-3-(2-hydroxyl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₇H₂₂O₅N₅)⁺, m/z =376.2.

### Example 19

A salt of Example 19 was specifically shown in formula (19) or formula (19'). That is, R¹ was sulfhydryl, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

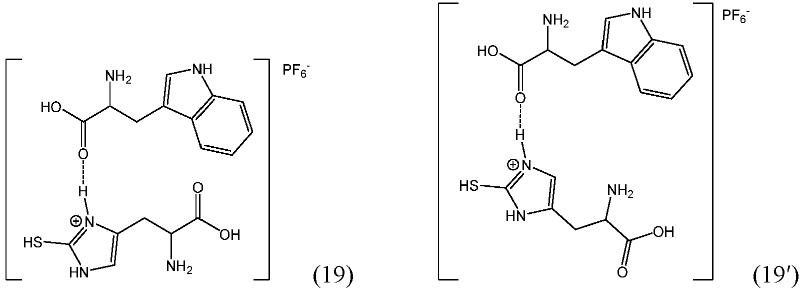

A method for preparing the salt in this example was as follows, in which:
- a first reactant was 2-amino-3-(2-sulfhydryl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₇H₂₂O₄N₅S)⁺, m/z =392.1.

### Example 20

A salt of Example 20 was specifically shown in formula (20) or formula (20'). That is, R¹ was amino, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

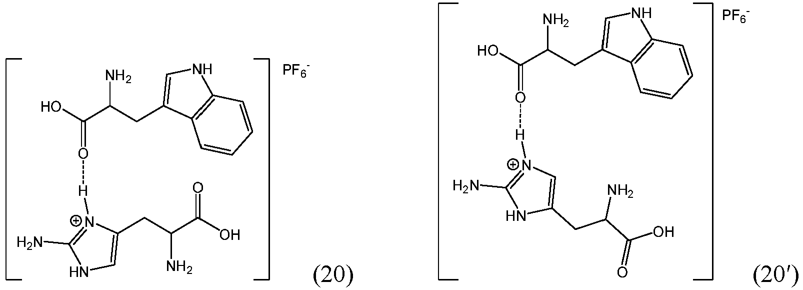

A method for preparing the salt in this example was as follows, in which:
- a first reactant was 2-amino-3-(2-amino-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₇H₂₃O₄N₆)⁺, m/z =375.2.

### Example 21

A salt of Example 21 was specifically shown in formula (21) or formula (21'). That is, R¹ was methyl, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

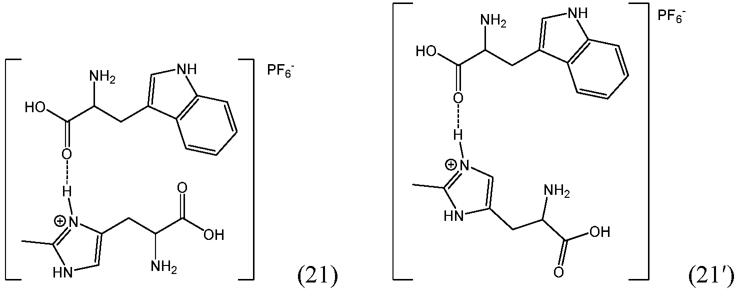

A method for preparing the salt in this example was as follows, in which:
- a first reactant was 2-amino-3-(2-methyl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₂O₄N₆)⁺, m/z =374.2.

### Example 22

A salt of Example 22 was specifically shown in formula (22) or formula (22'). That is, R¹ was phenyl, R² was hydrogen, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

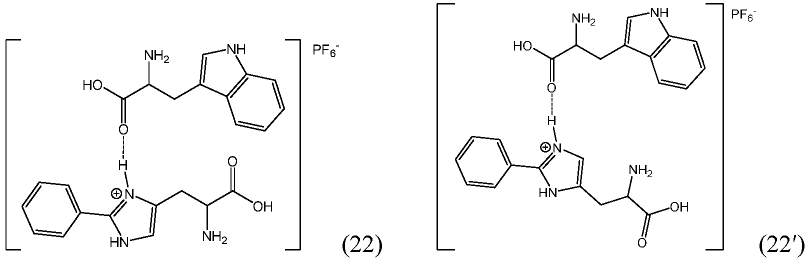

A method for preparing the salt in this example was as follows, in which:
- a first reactant was 2-amino-3-(2-phenyl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₂₃H₂₆O₄N₅)⁺, m/z =436.2.

### Example 23

A salt of Example 23 was specifically shown in formula (23). That is, R¹ was hydrogen, R² was methyl, R³ was hydrogen, R⁴ was hydrogen, R⁵ was amino, R⁶ was hydroxyl, R⁷was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

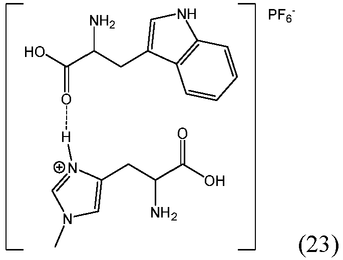

A method for preparing the salt in this example was as follows, in which:
- a first reactant was 2-amino-3-(1-methyl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄O₄N₅)⁺, m/z =374.2.

### Example 24

A salt of Example 24 was specifically shown in formula (24). That is, R¹ was hydrogen, R² was phenyl, R³ was hydrogen, R⁴ was hydrogen, R⁵ was amino, R⁶ was hydroxyl, R⁷was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

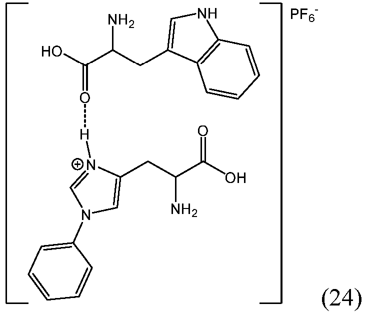

A method for preparing the salt in this example was as follows, in which:
- a first reactant was 2-amino-3-(1-phenyl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₂₃H₂₆O₄N₅)⁺, m/z =436.2.

### Example 25

A salt of Example 25 was specifically shown in formula (25). That is, R¹ was hydrogen, R² was triphenylmethyl, R³ was hydrogen, R⁴ was hydrogen, R⁵ was amino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

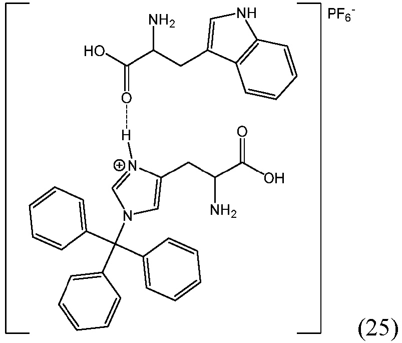

A method for preparing the salt in this example was as follows, in which:
- a first reactant was 2-amino-3-(1-triphenylmethyl-1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₃₆H₃₆O₄N₅)⁺, m/z =602.3.

### Example 26

A salt of Example 26 was specifically shown in formula (26) or formula (26'). That is, R¹ was hydrogen, R² was hydrogen, R³ was hydrogen, R⁴ was hydrogen, R⁵ was hydrogen, R⁶ was hydroxyl, R⁷was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

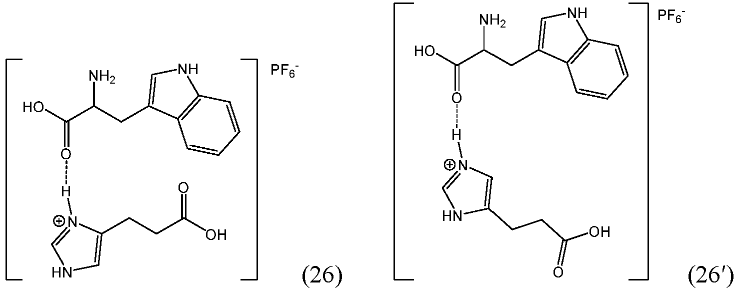

A method for preparing the salt in this example was as follows, in which:
- a first reactant was 3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₇H₂₁O₄N₄)⁺, m/z =345.2.

### Example 27

A salt of Example 27 was specifically shown in formula (27) or formula (27'). That is, R¹ was hydrogen, R² was hydrogen, R³ was hydrogen, R⁴ was hydrogen, R⁵ was methylamino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X as a hexafluorophosphate ion.

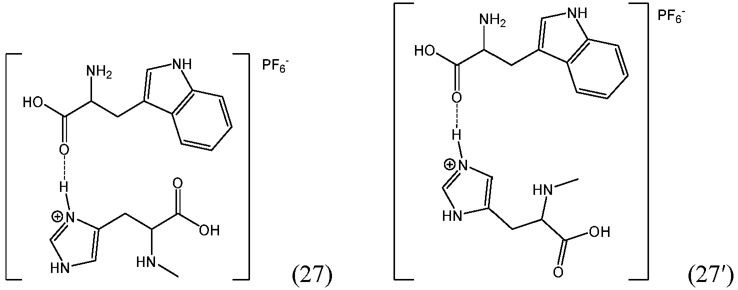

A method for preparing the salt in this example was as follows, in which:
- a first reactant was 2-methylamino-3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄O₄N₅)⁺, m/z =374.2.

### Example 28

A salt of Example 28 was specifically shown in formula (28) or formula (28'). That is, R¹ was hydrogen, R² was hydrogen, R³ was hydrogen, R⁴ was hydrogen, R⁵ was acetamido, R⁶ was hydroxyl, R⁷was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

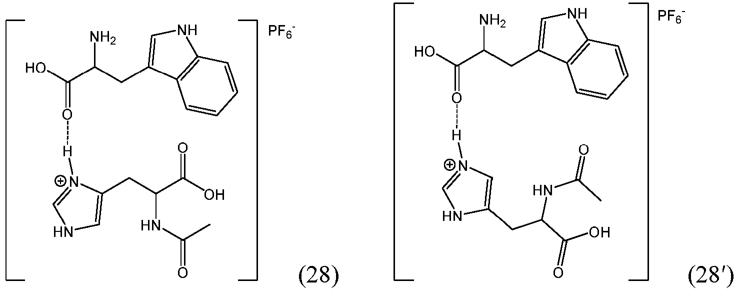

A method for preparing the salt in this example was as follows, in which:
- a first reactant was 2-acetamido-3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₉H₂₄O₅N₅)⁺, m/z =402.2.

### Example 29

A salt of Example 29 was specifically shown in formula (29) or formula (29'). That is, R¹ was hydrogen, R² was hydrogen, R³ was hydrogen, R⁴ was hydrogen, R⁵ was hydrazino, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

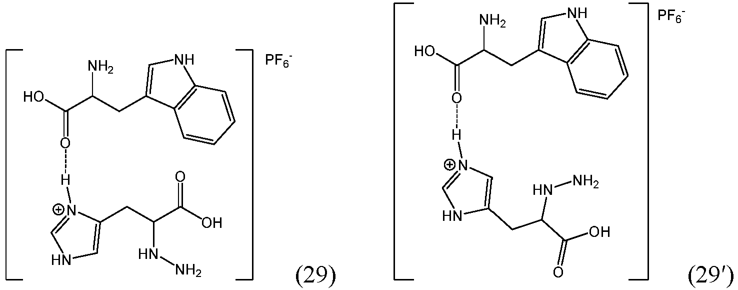

A method for preparing the salt in this example was as follows, in which:
- a first reactant was 2-hydrazino-3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₇H₂₃O₄N₆)⁺, m/z =375.2.

### Example 30

A salt of Example 30 was specifically shown in formula (30) or formula (30'). That is, R¹ was hydrogen, R² was hydrogen, R³ was hydrogen, R⁴ was hydrogen, R⁵ was pyrrolyl, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

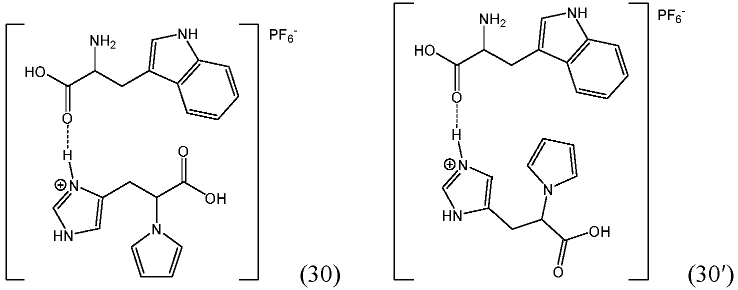

A method for preparing the salt in this example was as follows, in which:
- a first reactant was 2-(1*H*-pyrrol-1-yl)-3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₂₁H₂₄O₄N₅)⁺, m/z =410.2.

### Example 31

A salt of Example 31 was specifically shown in formula (31) or formula (31'). That is, R¹ was hydrogen, R² was hydrogen, R³ was hydrogen, R⁴ was hydrogen, R⁵ was phenylmethyl, R⁶ was hydroxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

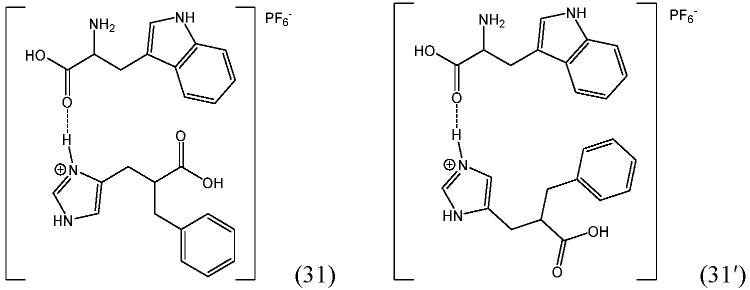

A method for preparing the salt in this example was as follows, in which:
- a first reactant was 2-phenylmethyl-3-(1*H*-imidazol-4-yl)propionic acid;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₂₄H₂₇O₄N₄)⁺, m/z =435.2.

### Example 32

A salt of Example 32 was specifically shown in formula (32) or formula (32'). That is, R¹ was hydrogen, R² was hydrogen, R³ was hydrogen, R⁴ was hydrogen, R⁵ was amino, R⁶ was methylamino, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

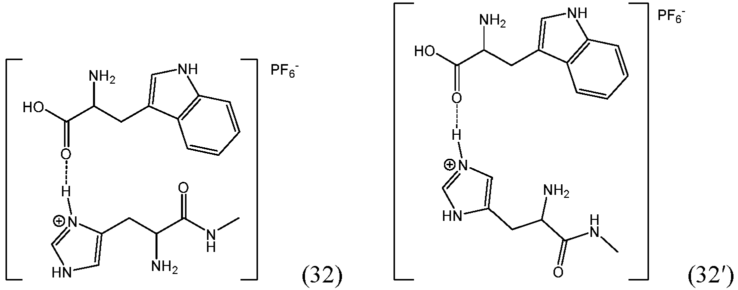

A method for preparing the salt in this example was as follows, in which:
- a first reactant was 2-amino-3-(1*H*-imidazol-4-yl)methylpropionamide;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₅O₃N₆)⁺, m/z =373.2.

### Example 33

A salt of Example 33 was specifically shown in formula (33) or formula (33'). That is, R¹ was hydrogen, R² was hydrogen, R³ was hydrogen, R⁴ was hydrogen, R⁵ was amino, R⁶ was methoxyl, R⁷ was -C(=O)-R⁶, Q¹ was hydrogen, Q² was hydrogen, Q³ was hydrogen, Q⁴ was hydrogen, Q⁵ was amino, Q⁶ was hydroxyl, *n* = 1, *m* = 1, *k =* 1, and X was a hexafluorophosphate ion.

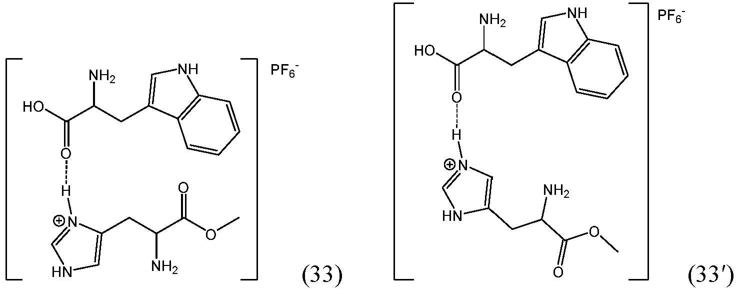

A method for preparing the salt in this example was as follows, in which:
- a first reactant was 2-amino-3-(1*H*-imidazol-4-yl)methylpropionate;
- a second reactant was 2-amino-3-(1*H*-imidazol-3-yl)propionic acid; and
- a first salt was ammonium hexafluorophosphate, i.e. a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, 4 g of first salt of this example was added, and the mixture was stirred at 25°C for 6 h to obtain a first aqueous solution comprising a protonated hydrochloride cation of the first reactant and the first anion.

0.89 g of second reactant was put into the first aqueous solution at 25°C and stirred gently for 24 h, to obtain a second aqueous solution comprising the cation of this example. The second aqueous solution was completely dialyzed with a dialysis bag with molecular weight cut-off of 200 Da, and then dried to obtain a first intermediate. The first intermediate was dissolved in 10 mL of ultrapure water, added with diluted ammonia water to adjust the pH to 9, and a large amount of beige precipitates were precipitated, followed by filtration; and the filter cake was washed twice with water and dried to obtain a product, i.e., the salt of this example.

### Detection of the product or the intermediate

The salt of this example was detected by using a high-resolution mass spectrometer, to obtain the cation of the present application, (C₁₈H₂₄O₄N₅)⁺, m/z =374.2.

### Experiments related to cell repair

### Reactive oxygen species scavenging experiments of PTIO models

The salts of Example 1, Example 4, and Example 18 were taken as compounds of experimental groups, and a mixture of histidine + tryptophan (a molar ratio of 1:1) was taken as a compound of control group. For each compound, different final concentrations were set, which were 0.025 g/mL, 0.05 g/mL, 0.1 g/mL, 0.2 g/mL, 0.4 g/mL, and 0.8 g/mL, respectively.

20 µL of each compound of experimental or control groups was added to 80 µL of 0.1 mg/mL PTIO aqueous solution to obtain an aqueous solution in which the final concentration of PTIO was 0.08 mg/mL, and the final concentration of the compound of experimental or control groups was as above. After the aqueous solution was shaken at 37°C for 1 h, the content of remaining PTIO was measured according to an absorbance at a wavelength of 560 nm.

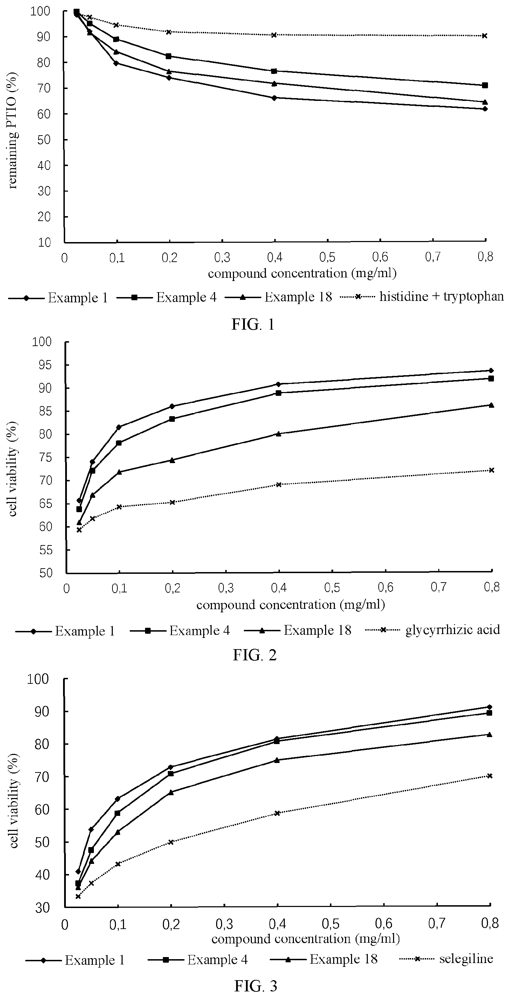

Results were shown in FIG. 1 and Table 1. The x-axis represented the concentration of the compound of experimental or control groups, the y-axis represented a concentration ratio of the normalized content of remaining PTIO, and the initial concentration content of PTIO was 100%. As shown in FIG. 1, when the concentration was at least 0.2 m/mL, the non-covalent dimer cation of the present application could scavenge reactive oxygen species represented by PTIO more efficiently compared to histidine and tryptophan monomers, and the scavenging efficiency was at least 3 to 4 times that of histidine and tryptophan monomers. Furthermore, with the increase of the concentration, the reactive oxygen species scavenging effect was improved.

**Table 1 Reactive oxygen species scavenging experiments of PTIO models**

| Normalized content of remaining PTIO Concentration ratio (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 98.6 | 92.1 | 79.6 | 74.0 | 66.0 | 61.4 |
| | Example 4 | 99.9 | 95.2 | 89.0 | 82.4 | 76.4 | 70.5 |
| | Example 18 | 99.5 | 91.6 | 84.1 | 76.4 | 71.6 | 64.0 |
| | Histidine + tryptophan 1:1 | 99.1 | 97.7 | 94.5 | 91.8 | 90.4 | 89.8 |

| | | | | | | | |
|---|---|---|---|---|---|---|---|
| Ethanol-induced damage models of gastric mucosal epithelial cells | | | | | | | |

Long-term alcoholics were prone to gastric mucosal damage due to long-term alcohol damage. In this experiment, a gastric mucosal epithelial cell damage model was established at a cellular level with ethanol, the salt of the present application or other compound of control group was/were added, and the technical effects of the non-covalent dimer cation of the present application in the gastric mucosal epithelial cell damage model were obtained.

The salts of Example 1, Example 4, and Example 18 were taken as compounds of experimental groups, and glycyrrhetinic acid was taken as a compound of control group. A blank control group (without ethanol) and a model group (with ethanol but without the salt or glycyrrhetinic acid in the examples) were set.

GES-1 human gastric mucosal epithelial cells were uniformly inoculated into a 96-well plate, about 5,000 cells per well. The cells were cultured in a complete RPMI 1640 medium for 6 h. After the cells were completely adhered to the wall, all the media were removed, 100 µL of cell culture solution, i.e., serum-free RPMI 1640 medium containing 7% of ethanol (a serum-free RPMI 1640 medium without ethanol was added to the blank group) was added to each well, and the cells were incubated and cultured for 12 h. After the cells were incubated in the ethanol for 12 h, the cell viability was significantly reduced to about 60%.

Then, all the media were removed again, 100 µL of each of serum-free RPMI 1640 media, i.e., cell culture solutions, containing 0.025 g/mL, 0.05 g/mL, 0.1 g/mL, 0.2 g/mL, 0.4 g/mL and 0.8 g/mL compound of experimental or control groups (a serum-free RPMI 1640 medium without ethanol was added to the blank group and the model group) were added to each well according to the groups, and the cells were cultured for 24 h.

Then, the cell viability was detected. Results of the model group showed that after the cells were cultured in a medium containing carbon tetrachloride for 12 h, the cell viability was significantly reduced to about 60%. Results of the experiment group and the control group were shown in FIG. 2 and Table 2. It can be seen that after the salt of the present application was added to the cell culture solution, the cell viability increased with increasing concentration, and the cell viability in the group of the salt of the present application was significantly higher than that of the glycyrrhetinic acid control group.

**Table 2 Cell viability of ethanol-induced damage models of gastric mucosal epithelial cells**

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 65.7 | 74.1 | 81.5 | 86.0 | 90.6 | 93.5 |
| | Example 4 | 63.9 | 72.2 | 78.1 | 83.3 | 88.7 | 91.7 |
| | Example 18 | 61.0 | 66.9 | 71.8 | 74.4 | 79.9 | 86.0 |
| | Glycyrrhetinic acid | 59.4 | 61.8 | 64.3 | 65.2 | 68.9 | 71.9 |

| | | | | | | | |
|---|---|---|---|---|---|---|---|
| Neurodegenerative disease-Parkinson's disease | | | | | | | |

6-Hydroxydopamine (6-OHDA) could be taken up by nerve cells, causing metabolic disorders of nerve cells and further inducing a Parkinson's disease model in vitro. In this experiment, a Parkinson's disease cell model was established at a cellular level with 6-OHDA, the salt of the present application or other compound of control group was/were added, and the technical effects of the non-covalent dimer cation of the present application in the Parkinson's disease cell model were obtained.

The salts of Example 1, Example 4, and Example 18 were taken as compounds of experimental groups, and selegiline was taken as a compound of control group. A blank control group (without 6-OHDA) and a model group (with 6-OHDA but without the salt of this example or selegiline) were set.

SH-SY5Y human neuroblastoma cells were uniformly inoculated into a 96-well plate, about 5,000 cells per well. The cells were cultured in a complete DMEM/F12 medium for 6 h. After the cells were completely adhered to the wall, all the media were removed, 100 µL of serum-free DMEM/F12 medium, i.e., a cell culture solution, containing 150 µmol/L of 6-OHDA (a serum-free DMEM/F12 medium without 6-OHDA was added to the blank group), was added to each well, and the cells were incubated and cultured for 24 h. After the cells were incubated in 6-OHDA for 12 h, the cell viability was significantly reduced to about 30%.

Then, all the media were removed again, 100 µL of each of serum-free DMEM/ F12 media, i.e., cell culture solutions, containing 0.025 g/mL, 0.05 g/mL, 0.1 g/mL, 0.2 g/mL, 0.4 g/mL and 0.8 g/mL of compound of experimental or control groups (a serum-free DMEM/ F12 medium without compound of experimental or control groups was added to the blank group and a model group) were added to each well according to the groups, and the cells were cultured in a cell culture solution for 24 h.

Then, the cell viability was detected. Results of the model group showed that after the cells were cultured in a medium containing 6-OHDA for 24 h, the cell viability was significantly reduced to about 30%. Results of the experiment group and the control group were shown in FIG. 3 and Table 3. It can be seen that after the salt of the present application was added to the cell culture solution, the cell viability increased with increasing concentration, and the cell viability in the group of the salt of the present application was significantly higher than that of the selegiline control group.

**Table 3 Cell viability of a Parkinson's disease model**

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 41.0 | 53.9 | 63.2 | 72.9 | 81.3 | 90.8 |
| | Example 4 | 37.4 | 47.6 | 58.8 | 70.8 | 80.6 | 89.0 |
| | Example 18 | 36.1 | 44.2 | 53.0 | 65.2 | 74.8 | 82.4 |
| | Selegiline | 33.4 | 37.5 | 43.3 | 49.9 | 58.5 | 69.8 |

| | | | | | | | |
|---|---|---|---|---|---|---|---|
| Neurodegenerative disease-Alzheimer's disease model | | | | | | | |

β-amyloid protein (Aβ) was often present in hippocampal neurons in Alzheimer's disease, causing damage to hippocampal neurons and thus leading to Alzheimer's disease. Therefore, in this experiment, an Alzheimer's disease cell model was established at a cellular level with Aβ(1-42) as one of subtypes of Aβ, the salt of the present application or other compound of control group was/were added, and the technical effects of the non-covalent dimer cation of the present application in the Alzheimer's disease cell model were obtained.

The salts of Example 1, Example 4, and Example 18 were taken as compounds of experimental groups, and ganglioside was taken as a compound of control group. A blank control group (without Aβ(1-42)) and a model group (with Aβ(1-42) but without the salt of this example or ganglioside) were set.

SH-SY5Y human neuroblastoma cells were uniformly inoculated into a 96-well plate, about 5,000 cells per well. The cells were cultured in a complete DMEM/F12 medium for 6 h. After the cells were completely adhered to the wall, all the media were removed, 100 µL of serum-free DMEM/F12 medium, i.e., the cell culture solution, containing 20 µmol/L of Aβ(1-42) (a serum-free DMEM/F12 medium without Aβ(1-42) was added to the blank group) was added to each well, and the cells were incubated and cultured for 24 h. After the cells were incubated in Aβ(1-42) for 48 h, the cell viability was significantly reduced to about 50%.

Then, all the media were removed again, 100 µL of each of serum-free DMEM/ F12 media, i.e., cell culture solutions, containing 0.025 g/mL, 0.05 g/mL, 0.1 g/mL, 0.2 g/mL, 0.4 g/mL and 0.8 g/mL of compound of experimental or control groups (a serum-free DMEM/ F12 medium without compound of experimental or control groups was added to the blank group and a model group) were added to each well according to the groups, and the cells were cultured in a cell culture solution for 24 h.

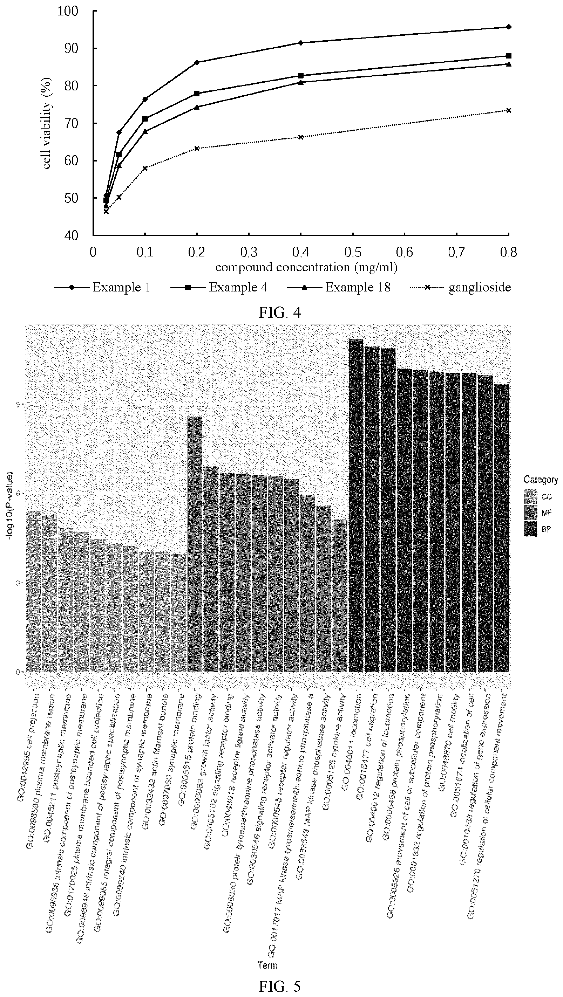

Then, the cell viability was detected. Results of the model group showed that after the cells were cultured in a medium containing Aβ(1-42) for 48 h, the cell viability was significantly reduced to about 50%. Results of the experiment group and the control group were shown in FIG. 4 and Table 4. It can be seen that after the salt of the present application was added to the cell culture solution, the cell viability increased with increasing concentration, and the cell viability in the group of the salt of the present application was significantly higher than that of the ganglioside control group.

FIG. 4 Cell viability of Alzheimer's disease model

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 50.7 | 67.5 | 76.4 | 86.2 | 91.5 | 95.7 |
| | Example 4 | 49.4 | 61.7 | 71.2 | 77.9 | 82.7 | 88.0 |
| | Example 18 | 48.0 | 58.7 | 67.8 | 74.3 | 80.9 | 85.8 |
| | Ganglioside | 46.4 | 50.3 | 58.0 | 63.2 | 66.3 | 73.5 |

### Transcriptomic analysis

Transcriptomic analysis was performed to explore how compounds work in cells.

Brain neuroma cells of a Neuro-2a mouse were evenly spread into two 10 cm dishes and cultured using a complete MEM medium. When the density reached 90%, all media were discarded, 10 mL of serum-free MEM medium was added, a solution of the salt of Example 1 was added to one of the dishes, and a final concentration was made to 0.1 mg/mL. An equal volume of PBS solution was added to the other dish. After 3 h of culture in a 37 °C cell incubator, all media were removed again, RNAs from the cells in the two dishes were extracted separately using Trizol, and transcriptomic analysis was performed.

In the transcriptomic results, GO enrichment analysis results of differentially expressed genes were classified according to molecular functions (MF), biological process (BP) and cellular components (CC), and the top 10 items with the smallest p-value in each classification, that is, the most significant enrichment, were selected for analysis. The results were shown in FIG. 5. It can be found that the cells treated with the salt of Example 1 had significant enhancement in terms of cell protection functions, protein binding abilities, growth factor activities, and cell signaling receptor binding activities, and were also accompanied by the improvement of cell migration abilities. The restoration of cell repair functions was promoted, and the cell repair level was improved.

### Experiments related to cell proliferation and wound repair

### Epidermal fibroblast proliferation experiments

Epidermal fibroblasts played a decisive role in epidermal wound repair, participated in the formation of connective tissues, and were related to the speed and effect of recovery from skin wounds. In this experiment, HFF-1 human epidermal fibroblasts were cultured, the salt of the present application or other compound of control group was/were added, and the technical effects of the non-covalent dimer cation of the present application in the proliferation of human epidermal fibroblasts were obtained.

The salts of Example 1, Example 4, and Example 18 were taken as compounds of experimental groups, and histidine + tryptophan (a molar ratio of 1:1) were taken as a compound of control group. HFF-1 human epidermal fibroblasts were uniformly inoculated into a 96-well plate, about 2000 cells per well. The cells were cultured in a complete DMEM medium for 6 h. After the cells were completely adhered to the wall, all the media were removed, 100 µL of each of complete DMEM media, i.e., cell culture solutions, containing 0.025 g/mL, 0.05 g/mL, 0.1 g/mL, 0.2 g/mL, 0.4 g/mL and 0.8 g/mL compounds of experimental groups (a complete DMEM medium without the compound of experimental groups was added to the blank group) was added to each well according to the groups, and the cells were cultured for 24 h.

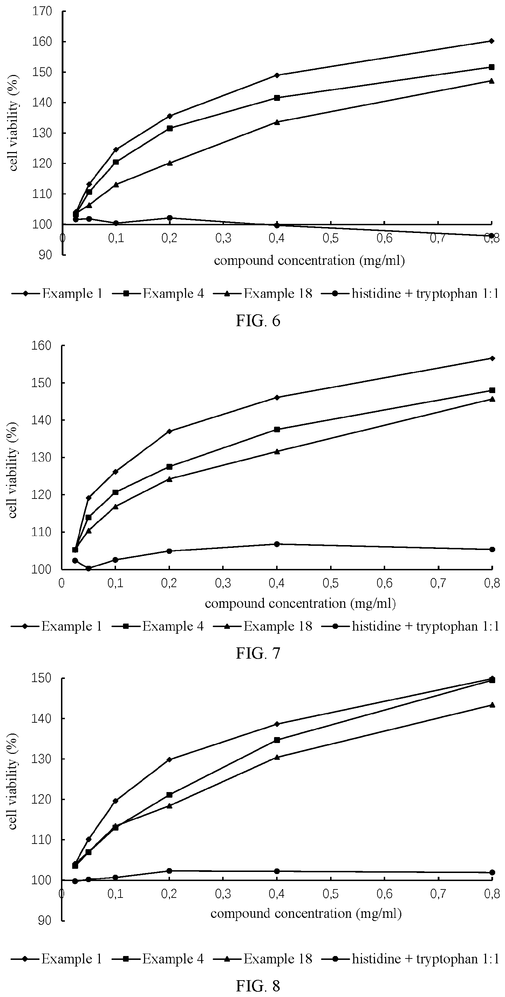

The cell viability was then detected, and the results were shown in FIG. 6 and Table 5. The results showed that the cell viability increased with the increase of concentration after the salt of the present application was added to the cell culture solution, which reflected the promotion of the growth and proliferation of human epidermal fibroblasts.

**Table 5 Cell viability in epidermal fibroblast proliferation experiments**

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 104.1 | 113.2 | 124.6 | 135.6 | 149.0 | 160.3 |
| | Example 4 | 103.4 | 110.7 | 120.5 | 131.6 | 141.6 | 151.7 |
| | Example 18 | 103.6 | 106.4 | 113.1 | 120.2 | 133.6 | 147.2 |
| | Histidine + tryptophan 1:1 | 101.7 | 101.9 | 100.5 | 102.2 | 99.7 | 96.3 |

### Keratinocyte proliferation experiments

Keratinocytes also played a key role in epidermal wound repair, and were involved in the repair of the stratum corneum on the skin surface, which was related to scar formation. In this experiment, HaCaT human immortalized keratinocytes were cultured, the salt of the present application or other compound of control group was/were added, and the technical effects of the non-covalent dimer cation of the present application in the proliferation of human immortalized keratinocytes were obtained.

The salts of Example 1, Example 4, and Example 18 were taken as compounds of experimental groups, and histidine + tryptophan (a molar ratio of 1:1) were taken as a compound of control group. HaCaT human immortalized keratinocytes were uniformly inoculated into a 96-well plate, about 2000 cells per well. The cells were cultured in a complete DMEM medium for 6 h. After the cells were completely adhered to the wall, all the media were removed, 100 µL of each of complete DMEM media, i.e., cell culture solutions, containing 0.025 g/mL, 0.05 g/mL, 0.1 g/mL, 0.2 g/mL, 0.4 g/mL and 0.8 g/mL compounds of experimental groups (a complete DMEM medium without the compound of experimental groups was added to the blank group) was added to each well according to the groups, and the cells were cultured for 24 h.

The cell viability was then detected, and the results were shown in FIG. 7 and Table 6. The results showed that the cell viability increased with the increase of concentration after the salt of the present application was added to the cell culture solution, which reflected the promotion of the growth and proliferation of human immortalized keratinocytes.

**Table 6 Cell viability in keratinocyte proliferation experiments**

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 105.2 | 119.2 | 126.2 | 137.0 | 146.1 | 156.6 |
| | Example 4 | 105.3 | 114.0 | 120.7 | 127.5 | 137.5 | 148.0 |
| | Example 18 | 105.4 | 110.4 | 116.9 | 124.2 | 131.6 | 145.7 |
| | Histidine + tryptophan 1:1 | 102.4 | 100.3 | 102.6 | 104.9 | 106.8 | 105.4 |

### Vascular endothelial cell proliferation experiments

Vascular endothelial cells were involved in the reconstruction of a vascular system and supported the wound healing during tissue repair. In this experiment, HUVEC human umbilical vein endothelial cells were cultured, the salt of the present application or other compound of control group was/were added, and the technical effects of the non-covalent dimer cation of the present application in the proliferation of human umbilical vein endothelial cells were obtained.

The salts of Example 1, Example 4, and Example 18 were taken as compounds of experimental groups, and histidine + tryptophan (a molar ratio of 1:1) were taken as a compound of control group. HUVEC human umbilical vein endothelial cells were uniformly inoculated into a 96-well plate, about 2000 cells per well. The cells were cultured in a complete vascular endothelium-dedicated medium for 6 h. After the cells were completely adhered to the wall, all the media were removed, 100 µL of each of complete vascular endothelium-dedicated media, i.e., cell culture solutions, containing 0.025 g/mL, 0.05 g/mL, 0.1 g/mL, 0.2 g/mL, 0.4 g/mL and 0.8 g/mL compounds of experimental groups (a complete vascular endothelium-dedicated medium without the compound of experimental groups was added to the blank group) was added to each well according to the groups, and the cells were cultured for 24 h.

The cell viability was then detected, and the results were shown in FIG. 8 and Table 7. The results showed that the cell viability increased with the increase of concentration after the salt of the present application was added to the cell culture solution, which reflected the promotion of the growth and proliferation of human umbilical vein endothelial cells.

**Table 7 Cell viability in vascular endothelial cell proliferation experiments**

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 104.1 | 110.1 | 119.6 | 129.8 | 138.6 | 149.9 |
| | Example 4 | 103.5 | 106.9 | 113.0 | 121.1 | 134.7 | 149.5 |
| | Example 18 | 104.0 | 107.0 | 113.4 | 118.4 | 130.4 | 143.4 |
| | Histidine + tryptophan 1:1 | 99.7 | 100.2 | 100.7 | 102.3 | 102.2 | 101.9 |

### Nerve cell proliferation experiments

Nerve cells participated in the reconstruction of a nervous system during tissue repair and played a decisive role in the health of the nervous system after the occurrence of a wound. In this experiment, nerve cells from CATH.a mice were cultured, the salt of the present application or other compound of control group was/were added, and the technical effects of the non-covalent dimer cation of the present application in the proliferation of nerve cells were obtained.

The salts of Example 1, Example 4, and Example 18 were taken as compounds of experimental groups, and histidine + tryptophan (a molar ratio of 1:1) were taken as a compound of control group. Nerve cells from CATH.a mice were uniformly inoculated into a 96-well plate, about 2000 cells per well. The cells were cultured in a complete RPMI 1640 medium for 6 h. After the cells were completely adhered to the wall, all the media were removed, 100 µL of each of complete RPMI 1640 media, i.e., cell culture solutions, containing 0.025 g/mL, 0.05 g/mL, 0.1 g/mL, 0.2 g/mL, 0.4 g/mL and 0.8 g/mL compounds of experimental groups (a complete RPMI 1640 medium without the compound of experimental groups was added into the blank group) was added to each well according to the groups, and the cells were cultured for 24 h.

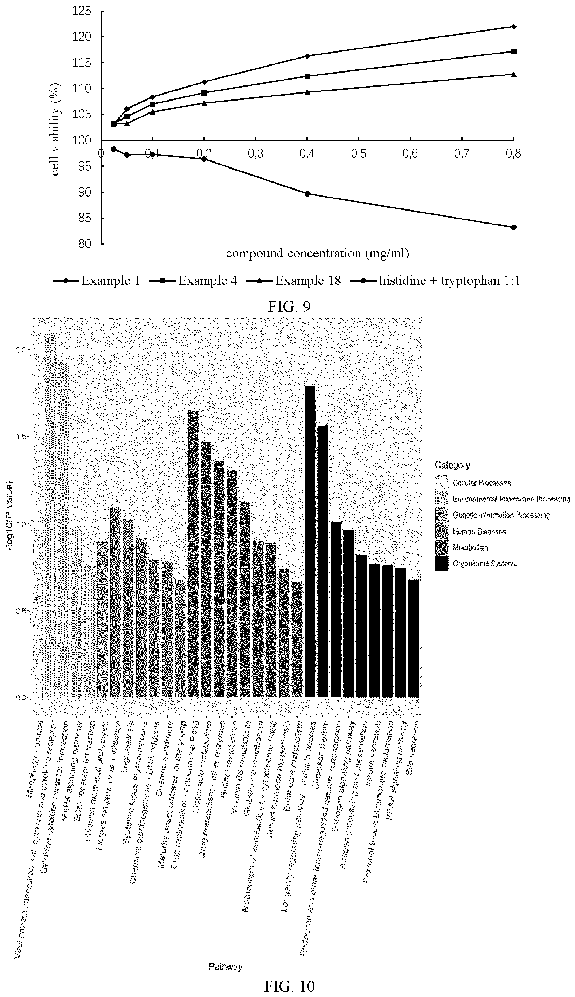

The cell viability was then detected, and the results were shown in FIG. 9 and Table 8. The results showed that the cell viability increased with the increase of concentration after the salt of the present application was added to the cell culture solution, which reflected the promotion of the growth and proliferation of nerve cells.

**Table 8 Cell viability in nerve cell proliferation experiments**

| Cell viability (%) | | Concentration of compound (mg/ml) | | | | | |
|---|---|---|---|---|---|---|---|
| | | 0.025 | 0.05 | 0.1 | 0.2 | 0.4 | 0.8 |
| Compound | Example 1 | 103.1 | 106.1 | 108.4 | 111.3 | 116.3 | 122.0 |
| | Example 4 | 103.3 | 104.6 | 107.0 | 109.2 | 112.4 | 117.2 |
| | Example 18 | 103.2 | 103.3 | 105.5 | 107.2 | 109.3 | 112.8 |
| | Histidine + tryptophan 1:1 | 98.3 | 97.2 | 97.3 | 96.4 | 89.7 | 83.2 |

### Transcriptomic analysis

Transcriptomic analysis was performed to explore how compounds exert the wound healing efficacy at a cellular level. HFF-1 human skin fibroblasts were evenly spread into two 10 cm dishes and cultured using a complete DMEM medium. When the density reached 90%, all media were discarded, 10 mL of serum-free DMEM medium was added, a solution of the salt of Example 1 was added to one of the dishes, and a final concentration was made to 0.1 mg/mL. An equal volume of PBS solution was added to the other dish. After 3 h of culture in a 37°C cell incubator, all media were removed again, RNAs from the cells in the two dishes were extracted separately using Trizol, and transcriptomic analysis was performed. The results were shown in FIG. 10.

In the transcriptomic results, KEGG enrichment analysis results of differentially expressed genes were subjected to KEGG classification according to cellular process CP, internal environmental information process EIP, gene information process GIP and metabolic process MP, and the top 10 items with the smallest p-value in each classification, that is, the most significant enrichment, were selected for analysis. It can be found that the activity of the cells treated with the salt of Example 1 in the functions of autophagy, growth factor receptor, DNA synthesis, cell metabolism regulation and the like was significantly enhanced, also accompanied by the improvement of cell migration ability. The regulation of cell proliferation was promoted, which in turn promoted tissue wound repair.

The above embodiments are only the preferred embodiments of the present application and cannot be intended to limit the protection scope of the present application, and any non-substantial changes and substitutions made by those skilled in the art on the basis of the present application also fall into the protection scope of the present application.

## Claims

1. A non-covalent dimer cation of formula (A), a tautomer thereof, or a stereoisomer thereof, 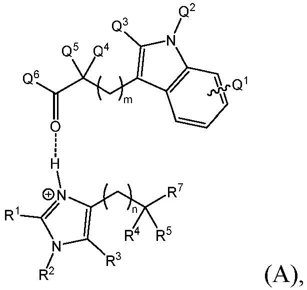 wherein,
bond "-------" in formula (A) is a non-covalent bond;
R⁷ is selected from a group consisting of hydrogen, hydroxymethyl, -C-R⁶, and -C(=O)-R⁶;
R¹ is selected from a group consisting of hydrogen, hydroxyl, sulfhydryl, amino, methyl, ethyl, and phenyl;
Q¹ is selected from a group consisting of hydrogen, hydroxyl, sulfhydryl, amino, methyl, ethyl, chloro, cyano, carboxyl, and phenyl;
R² and Q² are each independently selected from a group consisting of hydrogen, methyl, ethyl, propyl, phenyl, benzyloxymethyl, and triphenylmethyl;
R³ and Q³ are each independently selected from a group consisting of hydrogen, hydroxyl, sulfhydryl, amino, methyl, ethyl, and phenyl;
R⁴ and Q⁴ are each independently selected from a group consisting of hydrogen, methyl, ethyl, and propyl;
R⁵ and Q⁵ are each independently selected from a group consisting of hydrogen, benzyl, amino, methylamino, hydrazino, trimethylammonium, pyrrolyl, and amino further linked with a substituent derived from a C-terminal carbon atom of an amino acid, dipeptide or tripeptide;
R⁶ and Q⁶ are each independently selected from a group consisting of hydroxyl, deprotonated hydroxyl, methoxyl, methylamino, ethylamino, and a substituent derived from an N-terminal nitrogen atom of an amino acid, dipeptide or tripeptide; and
*m* and *n* are each independently selected from a group consisting of 0 and 1.

2. The cation according to claim 1, wherein
R⁴ is hydrogen or methyl;
R⁵ is selected from a group consisting of hydrogen, benzyl, amino, methylamino, hydrazino, pyrrolyl, and acetamido;
R⁶ is selected from a group consisting of hydroxyl, methoxyl, methylamino, and ethylamino; and
n is 1;
and/or
Q⁴ is hydrogen or methyl;
Q⁵ is selected from a group consisting of hydrogen, benzyl, amino, methylamino, hydrazino, pyrrolyl, and acetamido;
Q⁶ is selected from a group consisting of hydroxyl, methoxyl, methylamino, and ethylamino; and
m is 1.

3. The cation according to claim 1 or 2, wherein
R⁴ is hydrogen;
R⁵ is amino; and
R⁷ is -C(=O)-R⁶ and R⁶ is hydroxyl;
and/or
Q⁴ is hydrogen;
Q⁵ is amino; and
Q⁶ is hydroxyl.

4. The cation according to any one of claims 1 to 3, wherein
R¹ is hydrogen or hydroxyl;
R² is hydrogen; and
R³ is hydrogen;
and/or
Q¹ is hydrogen or hydroxyl;
Q² is hydrogen; and
Q³ is hydrogen;
and/or
R⁷ is -C(=O)-R⁶;
R² is the same as Q²;
R³ is the same as Q³;
R⁴ is the same as Q⁴;
R⁵ is the same as Q⁵; and
R⁶ is the same as Q⁶.

5. A salt, comprising:
the cation according to any one of claims 1 to 4; and
a first anion,
the first anion comprising at least one selected from a group consisting of a fluoride ion, a chloride ion, a bromide ion, an iodide ion, a sulfide ion, a nitrate ion, a sulfate ion, a sulfite ion, a thiosulfate ion, a persulfate ion, a selenate ion, a phosphate ion, a carbonate ion, a hexafluorophosphate ion, a hexafluorosilicate ion, an acetate ion, a sulfonate ion, a benzoate ion, and a polyphosphate ion.

6. The salt according to claim 5, wherein the first anion comprises at least one selected from a group consisting of a hexafluorophosphate ion, a hexafluorosilicate ion, a chloride ion, and a sulfate ion.

7. A method for preparing the cation according to any one of claims 1 to 4 or the salt according to claim 5 or 6, comprising:
contacting a first reactant with a first acid to obtain a first acid salt of the first reactant;
contacting the first acid salt of the first reactant with a first salt comprising a first anion in a first acidic condition, to obtain a first solution comprising a protonated first acid salt cation of the first reactant and the first anion;
adding a second reactant to the first solution in a second acidic condition, to obtain a second solution or a first intermediate; and
adding a first base to the second solution or contacting the first base with the first intermediate,
the first reactant comprising a compound of formula (C), and the second reactant comprising a compound of formula (D),

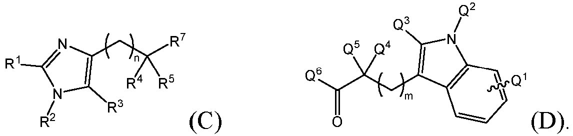

8. The method according to claim 7, wherein
the first acid is selected from a group consisting of hydrochloric acid, hydrobromic acid, and hydroiodic acid; and
the first salt consists of a first cation and the first anion, and the first cation is an ammonium ion;
and/or
the first reactant is contacted with the first acid at a first temperature, and the first temperature is 0°C to 70°C;
the first acid salt of the first reactant is contacted with the first salt at a second temperature, and the second temperature is 0°C to 70°C; and
the second reactant is added to the first solution at a third temperature, and the third temperature is 0°C to 70°C;
and/or
a molar ratio of the first reactant to the first acid is 1:2 to 1: 10;
a molar ratio of the first acid salt of the first reactant to the first salt is 1:2 to 1: 10; and
a molar ratio of a protonated first reactant cation to the second reactant is 1: 1 to 1:2;
and/or
a pH of the first acidic condition is less than or equal to 6; and
a pH of the second acidic condition is less than or equal to 6;
and/or
the first base comprises at least one selected from a group consisting of ammonia water, sodium hydroxide, potassium hydroxide, and triethanolamine; and
the first base is added to the second solution or contacted with the first intermediate to adjust the pH of the second solution or a solution comprising the first intermediate to be greater than 8.

9. Use of the cation according to any one of claims 1 to 4, or the salt according to claim 5 or 6, or the cation or salt prepared by the method according to claim 7 or 8 in antioxidation, preparation of an antioxidant, preparation of a drug for inhibiting, reducing or reversing oxidative stress in human or animal cells or for inhibiting, reducing or scavenging reactive oxygen species from human or animal bodies, or preparation of a drug for treating a disease or a symptom related to oxidative stress or reactive oxygen species;
optionally, the disease or symptom is selected from a group consisting of aging, inflammation, overweight, obesity, cancer, tumor, hepatopathy, neurodegenerative disease, arterial hypertension, atherosclerosis, cardiovascular disease, diabetes, hypercholesterolemia, chronic fatigue syndrome, ischemia reperfusion damage, neurodegenerative disease, ultraviolet-induced damage, and alcohol-induced damage;
optionally, the disease or symptom is Parkinson's disease or Alzheimer's disease; and
optionally, the disease or symptom is mucosal damage.

10. Use of the cation according to any one of claims 1 to 4, or the salt according to claim 5 or 6, or the cation or salt prepared by the method according to claim 7 or 8 in promotion of cell proliferation, or preparation of a drug for promoting cell proliferation or treating, alleviating or repairing a wound;
optionally, the cells are cells located in the skin;
optionally, the cells are selected from a group consisting of epidermal fibroblasts, keratinocytes, vascular endothelial cells, and nerve cells; and
optionally, the wound comprises a skin wound.
